(19) Europäisches Patentamt
European Patent Office
Office européen des brevets

(11) EP 4 778 462 A1

(12) EUROPEAN PATENT APPLICATION

(43) Date of publication:
22.07.2026 Bulletin 2026/30

(21) Application number: 26152433.4

(22) Date of filing: 16.01.2026

(51) International Patent Classification (IPC):
A61B 5/339 (2021.01)   A61B 5/00 (2006.01)

(52) Cooperative Patent Classification (CPC):
A61B 5/7435; A61B 5/339; A61B 5/7475;
A61B 2505/01; A61B 2560/0276

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH LA MA MD TN

(30) Priority: 17.01.2025  US 202563746890 P
15.01.2026  US 202619450663

(71) Applicant: Stryker Corporation
Portage, Michigan 49002 (US)

(72) Inventors:
• LINVILLE, David J.
Portage, MI 49002 (US)
• LIU, Michelle
Portage, MI 49002 (US)

(74) Representative: V.O.
P.O. Box 87930
2508 DH Den Haag (NL)

(54) **ELECTRONICALLY PRESENTING REAL-TIME AND HISTORICAL ELECTROCARDIOGRAMS**

(57) Techniques for electronic devices including medical devices and non-medical devices are described. An example method includes moving from a dynamic real-time snapshot of an ECG to a historical snapshot of the ECG; and generating the dynamic real-time snapshot of the ECG and the historical snapshot of the ECG, the historical snapshot of the ECG being selected from among a dynamic historical snapshot of the ECG or a static historical snapshot of the ECG. The dynamic real-time snapshot includes an ECG presented in an electronic format. The historical snapshot includes an ECG presented in a pseudo-paper format.

FIG. 1

**Description**

CROSS-REFERENCE TO RELATED APPLICATION

This application claims priority to and benefit of U.S. Provisional Patent Application No. 63/746,890 filed January 17, 2025. The entire contents of which are incorporated herein by reference in their entirety.

BACKGROUND

Electronic devices can output information in a variety of ways. Devices with screens generally display real-time data whether of the machine itself, sensor information, or an executed function. Some devices generate paper printouts with the data enabling users to view historical information from the device.

For example, an electrocardiogram (ECG) is indicative of the electrical activity of a heart over time. The electrical activity of an individual is identified, for instance, by measuring relative voltages between various electrodes placed on the body of the individual. Some medical devices (e.g., patient monitoring devices, patient treatment devices, and the like) display ECGs electronically, generally as a waveform. Some devices print ECG waveforms on paper. The paper printouts can enable caregivers to view historical ECG information, during and after administration of treatment.

BRIEF DESCRIPTION OF THE DRAWINGS

FIG. 1 illustrates an example environment in which a device is utilized to administer treatment, communicate with other devices, receive user input, and transition, via a user interface (UI), between presenting real-time electronic and historical pseudo-paper electrocardiograms in different formats.

FIG. 2 illustrates a device that transitions between real-time and historical electrocardiogram modes.

FIG. 3 illustrates a medical device that presents individual waveforms of an electrocardiogram (ECG) in electronic and pseudo-paper formats.

FIG. 4 illustrates an example environment in which a UI of a device receives user swipe or tap input to request the UI to pan or snap between a real-time and pseudo-paper electronic ECG.

FIG. 5 illustrates an example process for presenting a dynamic real-time snapshot of an ECG and moving from the dynamic real-time snapshot of the ECG to the historical snapshot of the ECG.

FIG. 6 illustrates an example process for presenting a snapshot of an ECG, updating the snapshot when the display is in a backtrack mode, and updating the snapshot when the display is in a snapback mode.

FIG. 7 illustrates an example environment in which a UI of a device receives user swipe or tap input to request the UI to pan or snap between a real-time and pseudo-paper electronic record of a capnogram.

FIG. 8 illustrates an example of an external defibrillator configured to perform various functions described herein.

DETAILED DESCRIPTION

Various implementations described herein relate to different modes and tools utilized to electronically present real-time data and historical data. Such data can be related to the functioning of a machine itself, for example, is it functioning as expected, glitching, or issuing error messages; related to the physical environment in which the machine is located, for example temperature, humidity, motion, or geographical location; and/or to the functions the machine is executing such as the collection of real-time and historical physiological parameter waveforms. Exemplary physiological parameter waveforms may include electrocardiogram (ECG), end-tidal $CO_2$ (EtCO$_2$), SPO$_2$ plethysmography, blood pressure, and patient temperature.

For example, an ECG is presented by a user interface (UI) of an electronic device. According to various cases, the modes include a real-time mode and a pseudo-paper mode. For instance, the real-time mode is utilized to present a portion of the ECG associated with a substantially current time period in an electronic format. In some cases, the pseudo-paper mode is utilized to present a portion of the ECG associated with a historical time period in a format that resembles a paper printout of the ECG. That is, the UI may display a snapshot of the ECG that resembles a paper printout of a portion of the ECG detected during a time interval. In some examples, the tools include a pan tool utilized to pan between the real-time mode and the pseudo-paper mode. In those examples, the tools include a snap tool utilized to snap between the real-time mode and the pseudo-paper mode. The UI, in some cases, pans between the real-time mode and the pseudo-paper mode, in response to detecting a swipe input to the UI. The UI, in some cases, snaps between the real-time mode and the pseudo-paper mode, in response to detecting a tap input to the UI.

According to some cases, the modes are dynamic or static. For instance, the real-time mode utilized to present the ECG is dynamic, such that a time period represented by the displayed ECG is continually updated. In some examples, the pseudo-paper mode utilized to present the ECG is dynamic, such that the device updates the represented time period by playing back the historical ECG. In some cases, the device operates in a static real-time or pseudo-paper mode, such that the ECG is displayed over a constant, unchanging time period. In some cases, for instance with the ECG being presented according to the dynamic real-time mode, a dynamic real-time snapshot of the ECG is presented. In some cases, for instance with the ECG being presented according to the static pseudo-paper mode, a static historical snapshot of the ECG is presented. In those or other cases, the static historical snapshot is separated from the dynamic real-time snapshot by a delay in time that is continually extending. Such modes and UI are applicable to other physiological waveform and machine information displays including end-tidal $CO_2$, $SPO_2$ plethysmography, blood pressure, and patient temperature, as well as information relating to the functioning or environmental conditions of the machine itself.

According to some examples, the UI receives selections via user input to the UI and switches between modes in response to receiving the selections. In some cases, a selection received via the swipe input to the UI is utilized by the UI to present the historical ECG in the pseudo-paper mode. For instance, the UI automatically transitions to the pseudo-paper mode to dynamically or statically present the historical ECG in response to receiving the swipe input. In some cases, a selection received via the tap input to the UI is utilized by the UI to present the ECG in the real-time mode. For instance, the UI automatically transitions to the real-time mode to dynamically present the ECG in response to receiving the tap input. In some cases, the selection utilized to present the ECG in the real-time mode is received via the tap input to a button presented by the UI. In those or other cases, the selection utilized to present the ECG in the real-time mode is received via the tap input to any portion of the UI, for example, when user equipment (UE) does not have an alternate input device such as a button. Similar interactions may be used with other physiological waveforms and data displays.

Electronic devices may use cumbersome and resource intensive paper printout operations to allow users to view historical information about the device, the environment within which the device is located, or the functions executed by the device. Various techniques discussed herein enable the electronic devices to replace paper printouts with pseudo-paper electronic physiological readings such as ECGs, end-tidal $CO_2$, $SPO_2$ plethysmography, blood pressure, and patient temperature. Paper printouts may also be replaced with pseudo-paper electronic displays of information related to machine functioning and environmental conditions.

For example, the electronic devices operate according to various modes for presenting various ECGs or other readings in different formats, including an electronic format and a pseudo-paper format. In some cases, the electronic devices switch between presenting the ECGs in the different formats in response to selections received via user input to the electronic devices. For instance, the user input, being simple and straightforward, enables the caregivers to intuitively switch between the real-time ECGs in the electronic format and the historical ECGs in the pseudo-paper format. In some cases, the electronic devices, refraining from performing paper printouts, efficiently, reliably, and quickly generate, present, and switch between the various ECGs. Similar switches may be performed to obtain other data such as data related to the machine's functioning, environmental data, or machine function execution such as acquisition of ECGs, end-tidal $CO_2$, $SPO_2$ plethysmography, blood pressure, and patient temperature.

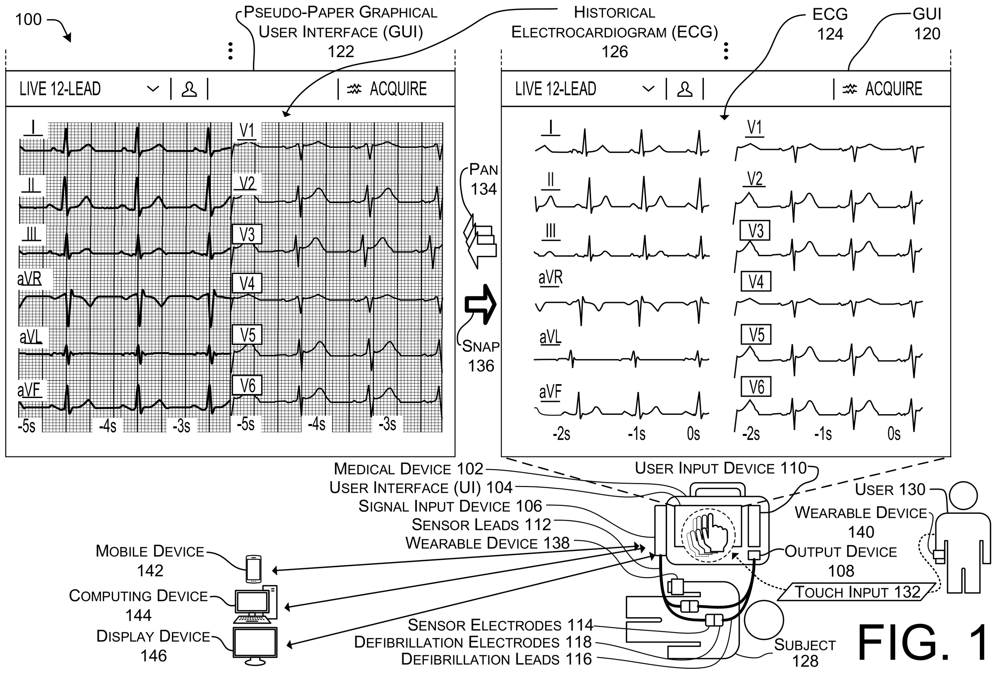

FIG. 1 illustrates an example environment 100 in which a device is utilized to administer treatment, communicate with other devices, receive user input, and pan and snap, via a user interface (UI), between real-time electronic and historical pseudo-paper electrocardiogram formats. As illustrated, the device includes an electronic device, such as a medical device 102; and the UI includes a UI 104. In various examples, the medical device 102 includes a signal input device 106, an output device 108, and a user input device 110. In some examples, the medical device 102 is couplable, such as via the signal input device 106, to sensor leads 112. In those or other examples, the medical device 102 is couplable to sensor electrodes 114, via the sensors leads 112. In those or other examples, the medical device 102 is couplable, such as via the output device 110, to defibrillation leads 116. In those or other examples, the medical device 102 is couplable to defibrillation electrodes 118, via the defibrillation leads 116.

In various cases, the medical device 102 is a defibrillator, though other devices such as a capnograph, sphygmomanometer, pulse oximeter, or mixed use devices may also be used. For example, the medical device 102 is an external defibrillator, such as an automated external defibrillator (AED) or a monitor-defibrillator. In some cases, the medical device 102 is a portable medical device.

In some examples, the UI 104 is a display, and/or is output a display of the medical device 102, is configured to graphically output physiological parameters. For instance, the UI 104 includes a graphical user interface (GUI) that includes at least a portion of physiological parameters, such as an ECG. In various cases, the GUI includes a GUI 120, a pseudo-paper GUI 122, one or more other GUIs, or any combination thereof. In some examples, the ECG includes an ECG 124, a historical ECG 126, one or more other types of ECGs, or any combination thereof. Other devices may display other readings such as $EtCO_2$, $SPO_2$ plethysmography, blood pressure, and patient temperature.

The UI 104 is configured to relay information from the medical device 102 to a user (e.g., the user 130, as discussed below in further detail). In some examples, the UI 104 includes a visual output device and/or auditory output device. For example, the visual output device includes a display and/or a touchscreen that outputs the information as a visual signal. In some cases, the touchscreen is included in and/or integrated with the touch interface included in the user input device 110 for the medical device 102. In some cases, the user inputs a signal to the medical device 102 via the touchscreen by selecting and/or manipulating items displayed on the touchscreen. The auditory output device includes, for example, a speaker that provides an audible signal that is heard by the user. Examples of the audible signal include instructions regarding treatment of a patient or other indications of patient data including physiological parameter values that are outside of a pre-determined range. The patient data is acquired and/or sensed by the medical device 102.

The UI 104 (e.g., and/or the user input device 110) includes various interfaces, such as a physical interface, an electronic interface, or combination thereof, to allow the user to interact with the medical device 102. Using the UI 104 (e.g., and/or the user input device 110), the user operates and/or accesses one or more features or systems of the medical device 102. For example, a cursor system, a touch interface, buttons, switches, or combination(s) thereof, are included on the medical device 108 as the UI 104 (e.g., and/or the user input device 110).

Exemplary waveforms are described as ECG or ECGs, though other waveforms or graphically mapped physiological parameter such as $EtCO_2$, $SPO_2$ plethysmography, blood pressure, and patient temperature or machine functioning data such as battery condition may also be displayed in the same or a similar manner. In some cases, individual ones of the ECG 124, the historical ECG 126, and/or the other ECG(s) include an axis (e.g., a first axis) corresponding to time and an axis (e.g., a second axis) corresponding to electrical signal magnitude (e.g., voltage). For instance, the first axis is horizontal. In such an instance or another instance, the second axis is vertical.

In some examples, the signal input device 106 detects physiological parameters of a subject 128. In some cases, the physiological parameters indicate a condition of a heart of the subject 128. According to various examples, the physiological parameters include an electrical signal output by the heart of the subject 128, an electrical impedance (e.g., transthoracic impedance) of the subject 128, a heart rate of the subject 128, a pulse rate of the subject 128, a blood oxygenation (e.g., pulse oxygenation, plethysmograph, etc.) of the subject 128, an airway parameter of the subject (e.g., capnograph, end-tidal $CO_2$, respiration rate, etc.), or any combination thereof. In some examples, the signal input device 106 includes one or more ports configured to receive analog signals indicative of the physiological parameters. The one or more ports may be electrically coupled to one or more sensors.

According to various examples, the physiological parameters detected by the signal input device 106 include an electrical signal output by the heart of the subject 128. In some cases, various cables, such as the sensor leads 112, are coupled between the signal input device 106 and the sensor electrodes 114. For instance, the sensor electrodes 114 are coupled to the subject 128. For instance, the sensor electrodes 114 are adhered to the subject 128 via an adhesive. In various examples, the sensor electrodes 114 receive an electrical signal (e.g., the voltage) output by the heart of the subject 128. In some cases, the signal input device 106 is configured to convert the electrical signal into digital data indicative of the physiological parameters. For instance, the digital data indicative of the physiological parameters includes digital data indicative of the ECG (also referred to as an "ECG signal"), including the ECG 124, the historical ECG 126, and/or the other ECG(s), of the subject 128. In some examples, the signal input device 106 generates the ECG (also referred to as an "ECG signal"), including the ECG 124, the historical ECG 126, and/or the other ECG(s) in response to receiving the electrical signals via the sensor electrodes 114. Although FIG. 1 illustrates a pair of sensor electrodes 114, in some examples, the signal input device 108 receives electrical signals indicative of the ECG 124, the historical ECG 126, and/or the other ECG(s), from any number of sensor electrodes 114 (e.g., two, three, or more sensor electrodes 114). For example, a 12-lead ECG is obtained from ten sensor electrodes 114 placed on the skin of the subject 128. In some cases, the signal input device 106 includes an analog to digital converter.

In various examples, the medical device 102 utilizes the output device 108, alternatively or additionally to, the UI 104, as discussed below in further detail. For instance, the medical device 102 is configured to output an indication of a mode (e.g., an active mode, a real-time mode, a backtrack mode, a historical mode, a pseudo-paper mode, a snapback mode, as discussed below in further detail, one or more other modes of various types, or any combination thereof), a state (e.g., a state of being powered on), or any combination thereof. For example, the medical device 102 identifies a mode in which the medical device 102 is operating and outputs an indication of the mode to a user (e.g., the user 130, as discussed below in further detail). In various implementations, the mode is adjusted by the user 130, the medical device 102 determines the mode corresponding to the adjusted mode, and/or outputs the indication of the adjusted mode. In various examples, the medical device 102 outputs the indication of the mode on the display UI 104. In some cases, the medical device 102 outputs the indication of the mode via the output device 108. The output device 108 includes, for example, a speaker configured to output audio indicative of the mode, a printer configured to print the ECG 124, the historical ECG 126, and/or the other ECG(s), possibly corresponding and/or according to the mode, or the like. Thus, the indication of the mode is output, in various implementations, as a visual signal, an auditory signal, a haptic signal (e.g., as vibration), or the like.

In some cases, the medical device 102 receives an input signal from the user input device 110 and outputs the ECG 124, the historical ECG 126, and/or the other ECG(s) in response to receiving the input signal. For instance, the user input device 110 identifies and/or receives one or more selections via user input from the user 130, and to the UI 104 and/or the user input device 110. In such an instance or another instance, the user input device 110 generates the input signal in response to receiving the selection(s). In some examples, the user input device 110 includes a keypad, a cursor control, a touch-sensitive display (e.g., the touchscreen), a voice input device, a haptic feedback device, a button, a track pads, a keyboard, or any combination thereof. In some cases, the user input device 110 includes touch sensors integrated with the UI 104, such that the user input device 110 senses the touch of the user 130 on the UI 104. For instance, the UI 104 is a touchscreen including one or more touch sensors configured to generate the input signal in response to receiving user input, such as a touch from the user 130. In some examples, touch input (e.g., the touch input 132, as discussed below in further detail, which includes one or more touches of various types) can be received, by the medical device 102, as the input signal (also referred to herein as "user input") from the user 130.

In various implementations, the medical device 102 is a defibrillator configured to output an electrotherapy signal, such as an electrical (e.g., defibrillating) shock and/or a pacing pulse to the subject 128. For example, the output device 108 is connected to a set of defibrillation leads, such as the defibrillation leads 116. In some cases, various cables, such as the defibrillation leads 116, are connected to the defibrillation electrodes 118 that are in contact with the skin of the subject 128. According to some examples, the defibrillation electrodes 118 are integrated with, or at least partially in contact with, at least some of the sensor electrodes 114. In some cases, the output device 108 includes an electrical circuit configured to selectively output an electrotherapy signal across the defibrillation electrodes 118. For example, the output device 108 includes a capacitor configured to store a voltage and a discharge circuit configured to discharge the voltage across the defibrillation electrodes 118. The voltage is applied over the heart of the subject 128 and depolarizes cells within the heart. The voltage may cause the heart to, at least eventually, restore a healthy heart rhythm.

The medical device 102, in some cases, selectively outputs the electrotherapy signal based on the ECG 124. For example, the medical device 102 outputs the defibrillation shock in response to detecting a shockable rhythm in the ECG 124. Shockable rhythms include ventricular fibrillation (VF) and ventricular tachycardia (VT). Optionally, the medical device 102 outputs a recommendation suggesting that the defibrillation shock should be applied to the subject 128. The recommendation is based on detecting the shockable rhythm in the ECG 124. In some cases, the medical device 102 outputs the defibrillation shock based on receiving an input signal, which may be input by the user 130 after the recommendation is output by the medical device 102. In various examples, the medical device 102 outputs the defibrillation shock at a particular time that depends on the ECG 124. For example, the medical device 102 outputs the defibrillation shock at a time in which the rate of change of the ECG 124 is positive. In some cases, the medical device outputs the defibrillation shock based on an input signal received from the user 130 via the user input device 110.

In some cases, the GUI 120 includes multiple plots corresponding to different (e.g., 12) waveforms of the ECG 124. For instance, a waveform represents and/or corresponds to a portion of the ECG). In various examples, the waveforms are generated by the medical device 102 (e.g., possibly by the signal input device 106) in response to receiving the electrical signals from the sensor electrodes 114, via the sensor leads 112. For instance, individual ones of the waveforms of the ECG 124 respectively correspond to ECG leads detected by the medical device 102. In such an instance or another instance, an ECG lead includes a graphical description of electrical activity of the heart. In various cases, the waveform of the ECG lead is created by analyzing the digital data generated by the signal input device 106.

In various cases, a number of sensor electrodes 114 (e.g., ECG electrodes utilized to generate the ECG) are in contact with the subject 128. For example, four electrodes 114 are in contact with limbs of the subject 128. For example, the electrodes 114 include a right arm (RA) electrode, a left arm (LA) electrode, a right leg (RL) or ground (G) electrode, and a left leg (LL) electrode. In addition, the electrodes 114 include precordial electrodes, including an electrode even with the fourth intercostal space (ICS) on the right margin of the sternum of the subject 128 (V1), an electrode even with the fourth ICS on the left margin of the sternum of the subject 128 (V2), an electrode even with the fifth ICS along the mid-clavicular line of the subject 128 (V4), an electrode placed between V2 and V4 (V3), an electrode even with the fifth ICS and an anterior axillary line of the subject 128 (V5), and an electrode even with the fifth ICS and a mid-axillary line of the subject 128 (V6). While 10 electrodes are described as an example, there may be any number of electrodes including 3, 5, 12, 15, 20, or more electrodes or any fraction thereof.

The medical device 102 is configured to detect relative voltage differences between various pairs of the electrodes 114. For example, although not illustrated in FIG. 1, the medical device 102 includes the signal input device 106, which is connected to the electrodes 114 by wired connections (e.g., the sensor leads 112). In particular, the medical device 102 detects the relative voltages of various combinations of the electrodes 114. These relative voltages can be referred to as the ECG leads. Graphical elements indicative of the leads (e.g., the waveforms) are displayed on the UI 104 of the medical device 102.

Lead I of the ECG 124 corresponds to the voltage of RA with respect to LA. Lead II of the ECG 124 corresponds to the voltage of RA with respect to the voltage of LL. Lead III of the ECG 124 corresponds to the voltage of LL with respect to the voltage of LA. Lead I, lead II, and lead III of the ECG 124 can be referred to as "Einthoven leads" herein, which correspond to relative voltages between the vertices of Einthoven's triangle. RL or G may be used as the ground for the measurements of lead I, lead II, and lead III of the ECG 124.

Other leads of the ECG 124 are unipolar leads representative of the voltage difference between a virtual electrode based other electrodes 114. A voltage of the virtual electrode, also known as Wilson's terminal ($V_W$), is defined according to Equation 1, shown below:

$$V_W = \frac{1}{3}(RA + LA + LL) \qquad\qquad (1)$$

Lead aVF, lead aVL, and lead -aVR of the ECG 124 are referred to as "augmented leads." Lead aVF of the ECG 124 corresponds to the voltage of LL relative to $V_W$. Lead aVL of the ECG 124 corresponds to the voltage of LA relative to $V_W$. Lead -aVR of the ECG 124 corresponds to the voltage of $V_W$ relative to RA. The Einthoven leads and the augmented leads are collectively referred to as "limb leads."

Leads V1-V6 of the ECG 124 are referred to as "precordial leads," and respectively correspond to the relative voltages between $V_W$ and the precordial electrodes. Lead V1 of the ECG 124 corresponds to the voltage of electrode V1 relative to $V_W$. Lead V2 of the ECG 124 corresponds to the voltage of electrode V2 relative to $V_W$. Lead V3 of the ECG 124 corresponds to the voltage of electrode V3 relative to $V_W$. Lead V4 of the ECG 124 corresponds to the voltage of electrode V4 relative to $V_W$. Lead V5 of the ECG 124 corresponds to the voltage of electrode V5 relative to $V_W$. Lead V6 of the ECG 124 corresponds to the voltage of electrode V6 relative to $V_W$.

Collectively, the twelve leads including lead I, lead II, lead III, lead aVF, lead aVL, lead -aVR, lead V1, lead V2, lead V3, lead V4, lead V5, and lead V6 of the ECG 124 are referred to as a "12-lead ECG." In some implementations, the medical device 102 detects a "15-lead ECG" that includes the twelve leads as well as three additional leads corresponding to the voltages of three posterior electrodes relative to $V_W$. The posterior electrodes include V7, V8, and V9. Electrode V7 is placed on a left posterior auxiliary line of the subject 128. V8 is placed on a tip of the left scapula of the subject 128. V9 is placed on a left paraspinal region of the subject 128. V7, V8, and V9 are all placed in the same horizontal plane as V6, or any other ECG configuration as would be known to one of ordinary skill in the art.

In various implementations, the medical device 102 presents, via the GUI 120, a snapshot of the ECG 124. For instance, the snapshot includes a dynamic real-time snapshot. In various examples, the ECG 124, and the waveforms therein, are shown and/or updated in real-time. For instance, a point on a right-most portion of each of the waveforms of the ECG 124 represents an ECG value at a current time.

In various cases, the medical device 102 operates in the active mode to present the dynamic real-time snapshot of the ECG 124. For instance, the active mode, being a real-time mode, includes the snapshot of the ECG 124 being presented dynamically, in real-time.

In some examples, the dynamic real-time snapshot of the ECG 124 is presented, in a real-time format, by the medical device 102 operating in the real-time mode. For instance, the real-time format includes an electronic format of the ECG 124.

In some cases, the medical device 102 identifies one or more touches from among the user input. For instance, the medical device 102 identifies, via the one or more touches, touch input 132. In various examples, the touch input 132 includes a swipe input received by the UI 104 and from the user 130. For instance, the touch input 132, such as the swipe input, is received to any location on the UI 104 (e.g., on the GUI 120).

In some cases, the medical device 102 (e.g., the GUI 120) updates the ECG 124 in response to receiving the swipe input, as the touch input 132. For instance, updating the ECG 124 includes moving the ECG 124. In such an instance or another instance, the UI 104 moves the ECG 124 to a historical snapshot of the ECG 124, such as the historical ECG 126. In various examples, the historical ECG 126 is the same as, and/or generated identically to, the ECG 124, except at a previous point in time.

In some examples, the historical ECG 126 includes a dynamic historical ECG or a static historical ECG. For instance, the dynamic historical snapshot of the ECG is separated from the dynamic real-time snapshot of the ECG 124 by a delay in time that is fixed. In such an instance or another instance, the static historical snapshot of the ECG is separated from the dynamic real-time snapshot of the ECG by a delay in time that is continually extending. In some cases, in response to the medical device 102 identifying a command (e.g., generated by the medical device 102 receiving the swipe input), the UI 104 scrolls from the dynamic real-time snapshot of the ECG to the dynamic historical snapshot of the ECG. In various examples, the medical device 102 generates, as the historical ECG 126 being the dynamic historical snapshot of the ECG, a dynamic 12-lead historical snapshot of the ECG. In some cases, the UI 104 is further configured to move from the ECG 124 (e.g., a dynamic real-time snapshot of the ECG) to the historical ECG 126 (e.g., the dynamic 12-lead historical snapshot of the ECG). In some cases, the medical device 102 generates, as the historical ECG 126 being the static historical snapshot of the ECG, a pseudo-paper snapshot that is static and includes historical data. For example, the medical device 102 moves (e.g. pans) from the historical ECG 126 being the dynamic historical snapshot of the ECG to the historical ECG 126 being the pseudo-paper snapshot of the ECG (e.g., beginning captured at an earlier or later time than a beginning of the dynamic real-time snapshot). While dynamic 12-lead historical snapshots are provided as an example, other numbers of leads such as 3, 5, and 15 may also be used.

In various cases, the medical device 102 moves between any of the ECG 124 being a dynamic snapshot, the historical ECG 126 being the dynamic historical snapshot, or the historical ECG 126 being the static historical snapshot to any other of the ECG 124 being a dynamic snapshot, the historical ECG 126 being the dynamic historical snapshot, or the historical ECG 126 being the static historical snapshot. For instance, moving between any of the ECGs to any other of the ECGs includes (e.g., such as according to device settings, as discussed below in further detail) moving via a pan, a jump, etc., or any other type of move.

In some cases, prior to generating a snapshot that includes a current snapshot, the medical device 102 generates an initial snapshot of the ECG, and an initial timestamp linked to initial data in the initial snapshot of the ECG (e.g., a snapshot of the historical ECG 126). In various cases, the initial timestamp identified a beginning of presenting a dynamic snapshot of any of the ECG 124 or the historical ECG 126. In various examples, the medical device 102 generates a current timestamp linked to current data in the current snapshot of the ECG (e.g., a current timestamp represents current data presented in the dynamic snapshot of the ECG 124). In some cases, the medical device 102 updates the current snapshot of the ECG to be the an initial snapshot of the ECG (e.g., the historical ECG 126) when the display is in the backtrack mode. In those or other cases, the medical device 102 presents, when the UI 104 is in a historical mode, the initial snapshot of the ECG (e.g., the historical ECG 126) to which the initial data is linked.

The ECG, such as the ECG 124, the historical ECG 126, and/or the other ECG(s), are moved by the UI 104 in various ways. In some examples, the UI 104 moves to the historical ECG 126 by the medical device 102 performing a pan operation (also simply referred to herein as "pan") 134. In those or other examples, the UI 104 moves from the ECG 124 to the historical ECG 126 by the medical device 102 performing the pan 134.

For instance, the ECG 124 is moved to the historical ECG 126 via the pan 134 in response to receiving the swipe input as the touch input 132. In such an instance or another instance, the ECG 124 is moved to the historical ECG 126 via the pan 134 in response identifying that a type of the touch input 132 is the swipe input, from among various types of input. In some examples, the medical device 102 identifies, selects, and/or presents the historical ECG 126 by identifying a current time, a time at which the historical ECG 126 was captured, an amount of time between the current time and the time at which the historical ECG 126 was captured, one or more other times of different types, or any combination thereof.

In some cases, the medical device 102 utilizes the pan 134 to move smoothly, without interruption, between the ECG 124 and the historical ECG 126. For example, the medical device 102 operates in the backtrack mode to move, via the pan 134, between the ECG 124, the historical ECG 126, and/or one or more other historical ECGs. In various cases, the pan 134 causes the medical device 102 to scroll from the ECG 124 to the historical ECG 126.

The ECG 124 and/or the historical ECG 126 are presented by the UI 104 in various ways. In some examples, the ECG 124, such as one or more waveforms therein, is presented via the GUI 120 via the electronic format. For instance, the electronic format is different from a format in which the historical ECG 126 is presented.

In some examples, the historical ECG 126, such as one or more waveforms therein, is presented via the pseudo-paper GUI 122 via a pseudo-paper format. For instance, the medical device 102 presents the historical ECG 126 by operating in the historical mode (e.g., and/or in the pseudo-paper mode). In such an instance or another instance, the medical device 102 presents the historical ECG 126 via the pseudo-paper GUI 122 by operating in the pseudo-paper mode (e.g., and/or in the historical mode).

In some cases, the pseudo-paper format includes a grid that represents the scale of time and voltage with respect to a pseudo-paper printout of the historical ECG 126. For example, the pseudo-paper format is utilized to present the historical ECG 126 with characteristics of a paper print-out (e.g., a grid, a color, etc.). In some cases, the gridlines are separated by distances corresponding to a scale of the grid. For instance, the grid facilitates a visual comparison between the length (e.g., duration) of one instance of a feature to another instance of a feature in the historical ECG 126. In various examples, the grid has a predetermined or selected scale. For example, each box of the grid has a predetermined width and/or a width that corresponds to a predetermined time interval. In some cases, each box of the grid has a predetermined height and/or a height that corresponds to a predetermined voltage. Examples of the feature include, for instance, a QRS complex, a PR interval (also referred to as a "PR segment"), a PR segment, an ST segment, a QT interval, or an RR interval of the electronic ECG. In some cases, any number of features are identified and/or identifiable for the historical ECG 126.

In various cases, the medical device 102 operates in the pseudo-paper mode and/or the historical mode to enable the user 130 to easily view the historical ECG 126. For instance, the historical ECG 126 is presented by the medical device 102 as a possible substitute for paper printouts. In some cases, the user 130 utilizes the pseudo-paper mode by customizing the touch input 132 to request the medical device 102 to present a desired past ECG. For instance, the user 130 quickly and easily pans and/or scrolls back the UI 104 to view the past ECG and/or any of the past waveforms.

The pan 134 is utilized to change the waveforms of the ECG 124 from being presented in the electronic format to being displayed to the pseudo-paper format. In some examples, the pan 134 is utilized to change from presenting, in the electronic format, all portions of the GUI 120 to presenting, in the pseudo-paper format, all portions of the pseudo-paper GUI 122. Alternatively, the pan 134 is utilized to individually change from presenting, in the electronic format, individual waveforms of the ECG 124 of the GUI 120 to presenting, in the pseudo-paper format, individual waveforms of the historical ECG 126 in the pseudo-paper GUI 122.

In various cases, the medical device 102 utilizes the pan 134 to change from presenting, in the electronic format, a particular waveform of the ECG 124 of the GUI 120 to presenting, in the pseudo-paper format, a particular waveform of the historical ECG 126 in the pseudo-paper GUI 122, in response to identifying that the touch input 132 (e.g., a start location, an end location, etc., of the swipe input, as discussed below in further detail) includes a swipe of the particular waveform.

For example, the swipe of the particular waveform includes a swipe (e.g., the start location, the end location, etc.) that overlaps with the waveform or, possible, a geometric shape (e.g., visible or not visible) outlining in the waveform.

Transitioning between any portion of the GUI 120 to a corresponding portion of the pseudo-paper GUI 122 occurs in any of various ways. For instance, changing between the electronic format to the pseudo-paper format includes gradually transitioning from any portion of the GUI 120 to the corresponding portion of the pseudo-paper GUI 122. In some cases, transitioning includes an instant change from any portion of the GUI 120 to any corresponding portion of the pseudo-paper GUI 122. Alternatively of additionally, transitioning includes fading out any portion of the GUI 120 and fading in the corresponding portion of the pseudo-paper GUI 122. Alternatively of additionally, transitioning includes any portion of the GUI 120 being overlapped (e.g., partially or entirely overlapped) by the corresponding portion of the pseudo-paper GUI 122, or vice versa.

In some cases, a portion of the GUI 120 utilized to present any of the waveforms in the ECG 124 is transitioned to an appropriate portion of the pseudo-paper GUI 122 utilized to present any corresponding waveform in the historical ECG 126. By way of example, a portion of the GUI 120 utilized to present a waveform (or "V1 waveform") associated with the V1 electrode in the ECG 124 is transitioned to a corresponding portion of the pseudo-paper GUI 122 utilized to present the V1 waveform in the historical ECG 126. For instance, the portion of the GUI 120 is transitioned to the corresponding portion of the GUI 122 by transitioning from a left side of the V1 waveform in the ECG 124 and to a right side of the V1 waveform in the historical ECG 126. In some cases, the transitioning from the left side of the V1 waveform in the ECG 124 and to the right side of the V1 waveform in the historical ECG 126 includes an instant change, a fade therebetween, a partial overlap therebetween, or any other type of transitioning.

In various examples, such as with respect to any of the techniques for moving between ECGs as discussed throughout this disclosure, all portions of the ECG 124 and/or the GUI 120 are transitioned to all of the corresponding portions of the historical ECG 126 and/or the pseudo-paper GUI 122 in unison (e.g., simultaneously, at the same speed/amount, in the same or different ways, or any combination thereof). For instance, all of the waveforms of the ECG 124 are transitioned to all of the corresponding waveforms of the historical ECG 126 in unison (e.g., simultaneously, at the same speed/amount, in the same or different ways, or any combination thereof).

For example, the instant change includes, during the pan 134, an instant change from the left side of the V1 waveform in the ECG 124 and to the right side of the V1 waveform in the historical ECG 126. In some cases, the fade includes, during the pan 134, a fade from the left side of the V1 waveform in the ECG 124 and to the right side of the V1 waveform in the historical ECG 126. In some cases, the partial overlap includes, during the pan 134, a partial overlap of the left side of the V1 waveform in the ECG 124, and the right side of the V1 waveform in the historical ECG 126. In various cases, transitioning between any waveforms of the ECG 124 and/or any portion of the GUI 120 occurs utilizing any of the techniques as discussed above for the V1 waveform.

The ECG 124 moving to the historical ECG 126 occurs in any of various ways in response to identifying any of various characteristics of the swipe input received as the touch input 132. In some examples, the characteristics of the swipe input include a length, a speed, a location (e.g., a start location, an end location, and/or one or more other locations), a curvature and/or an arc, etc., or any combination thereof. In those or other examples, the characteristics of the swipe input are utilized to identify characteristics of the historical ECG 126. In some cases, the characteristics of the historical ECG 126 include a time at which the historical ECG 126 was captured, whether the historical ECG 126 is static or dynamic, how long the historical ECG 126 is presented, one or more portions of the ECG 124 being moved to one or more portions the historical ECG 126, and so on, or any combination thereof.

For instance, the length includes a distance between any of one or more locations (e.g., a start location) (or "first location") and another of one or more locations (e.g., an end location) (or "second location"). In some cases, any of the locations is associated with one or more pixels (e.g., one or more pixel identifiers) and/or one or more x-y coordinates of the UI 104. For instance, a location (e.g., the first location and/or the second location) is identified as a location associated with x-y coordinates identifying a pixel, a location associated with groups of x-y coordinates identifying a group of corresponding pixels of the UI 104, any of various other locations of different types, or any combination thereof).

In some examples, a time associated with the historical ECG 126 is determined in response to identifying the length of the swipe input. For instance, according to a possible default setting of medical device settings (also referred to herein simply as "device settings" or "settings") associated with the medical device 102, the length of the swipe input is utilized to identify how far back in time to scroll from the ECG 124 and to the historical ECG 126.

In various cases, to determine how far back to scroll, the medical device 102 identifies an amount of time corresponding to the relative length and/or speed of the swipe input. For instance, the amount of time is utilized to determine the time interval associated with, and utilized to select, the historical ECG 126 to be displayed. In various cases, the time interval associated with, and utilized to select, the historical ECG 126 is identified by subtracting the amount of time (corresponding to the relative length and/or speed of the swipe input) from a current time (e.g., associated with the ECG 124).

In various examples, the amount of time utilized to select the historical ECG 126 is identified based on a swipe input length. In some cases, the amount of time is identified from among various amounts of time being predetermined as being linked to corresponding swipe input lengths. For instance, a relatively longer amount of time corresponds to a relatively longer length of the swipe input. In various cases, a historical ECG that was captured at the time separate from the current time by the amount of time is identified. The identified historical ECG is selected as the historical ECG 126.

In some instances, the speed associated with the swipe input is utilized to move from the ECG 124 to the historical ECG 126, such as by identifying whether the historical ECG 126 is dynamic or static. For instance, according to a possible default setting of medical device settings, the historical ECG 126 is presented as a dynamic ECG by identifying that a speed associated with the swipe input received as the touch input 132 is above a threshold speed. In those or other examples, the historical ECG 126 is presented as a static ECG by identifying that a speed, such as a speed, associated with the swipe input received as the touch input 132 is less than a threshold speed. For instance, a relatively faster speed of the swipe input corresponds to a dynamic ECG; and a relatively slower speed of the swipe input corresponds to a static ECG. In various cases, a historical ECG that is dynamic or static is selected as the historical ECG 126 by identifying that the swipe input is faster or slower, respectively. In some examples, according to a possible default setting of medical device settings, the speed utilized to identify that the historical ECG 126 is dynamic or static includes an average speed. In those or other examples, the speed utilized to identify that the historical ECG 126 is dynamic or static includes a maximum speed, a minimum speed, or any of any other speeds of various types.

Various locations are utilized to identify whether or not to disregard the swipe input. For instance, locations utilized to identify the swipe input as the touch input 132 include locations of the UI 104 (e.g., locations of the GUI 120, the ECG 124, and/or one or more other locations). In some examples, the start location, the end location, and/or one or more other locations of the UI 104 are utilized identify the swipe input as the touch input 132. In various cases, according to a possible default setting of medical device settings, any location of the UI 104 being identified as being associated with the swipe input it utilized to identify the swipe input as the touch input 132.

In some examples, any location of the UI 104 being identified as being associated with the swipe input is utilized to determine to move the ECG 124 to the historical ECG 126, via the pan 134. In those or other examples, a size of a distance in a horizontal direction (e.g., in an x-direction, along a horizontal-axis or x-axis) between the start location and the end location of the UI 104 is utilized to determine to utilize the pan 134 to move to the historical ECG 126. For instance, if the medical device 102 determines that the start location and the end location are separated (e.g., in the horizontal direction) by a distance that is above a threshold distance, the medical device 102 determines to move the ECG 124 to the historical ECG 126, via the pan 134. In such an instance or another instance, the start location being to a right side of the end location, is utilized to determine to move the ECG 124 to the historical ECG 126, via the pan 134. For example, an x-value in an x-y coordinate associated with the start location being greater than an x-value in an x-y coordinate associated with the end location, is utilized to determine to move the ECG 124 to the historical ECG 126, via the pan 134.

In some cases, the swipe input not satisfying conditions (e.g., the start location and the end location being separated in the horizontal direction by the distance that is above the threshold distance, the x-value in the x-y coordinate associated with the start location being greater than the x-value in the x-y coordinate associated with the end location) is disregarded. For instance, the medical device 102 disregarding the swipe input includes refraining from moving any portion of the UI 104 (e.g., the ECG 124 and/or the GUI 120).

Various curvatures and/or arcs associated with the swipe input are utilized to identify how much of the UI 104 (e.g., what portion of the UI 104) is utilized for the pan 134. For instance, according to a possible default setting of medical device settings, the medical device 102 utilizes any type of curvatures and/or any type of arcs associated with the swipe input to determine to move all (e.g., the entire portion) of the ECG 124 to all (e.g., the entire portion) of the historical ECG 126. In such an instance or another instance, in response to determining to move all (e.g., the entire portion) of the ECG 124 to all (e.g., the entire portion) of the historical ECG 126, the medical device 102 moves, via the UI 104, all (e.g., the entire portion) of the ECG 124 to all (e.g., the entire portion) of the historical ECG 126. In various cases, the medical device 102, determining to move all (e.g., the entire portion) of the ECG 124 to all (e.g., the entire portion) of the historical ECG 126, disregards any curvature and/or any arc associate with the swipe input.

In some instances, a curvature and/or an arc associated with the swipe input is utilized to determine to transition a portion (e.g., a partial portion) of the ECG 124 to a portion (e.g., a partial portion) of the historical ECG 126. For instance, the curvature and/or the arc having a degree of curvature that is above a threshold degree is utilized to determine to transition a portion (e.g., a partial portion) of the ECG 124 to a portion (e.g., a partial portion) of the historical ECG 126. In some cases, the degree of curvature is a central angle to ends of designated lengths of the arc. Alternatively, in some instances, the curvature and/or the arc associated with the swipe input is utilized to determine to transition all (e.g., an entire portion) of the ECG 124 is utilized to determine to all (e.g., an entire portion) of the historical ECG 126. For instance, the curvature and/or the arc having a degree of curvature that is less than a threshold degree is utilized to determine to transition all (e.g., an entire portion) of the ECG 124 is utilized to determine to all (e.g., an entire portion) of the historical ECG 126.

In some examples, the curvature includes an amount by which a curve of the swipe input (e.g., a curve of a smooth line approximation or a linear approximation of the swipe input or a portion, such as a largest portion, of the swipe input) is turning. For instance, the curvature includes a magnitude of a derivative of a unit tangent vector function with respect to an arc length of the curve. In various examples the arc includes a portion of a boundary of the curve (e.g., the curve of the smooth line approximation or the linear approximation of the swipe input or a portion, such as a largest portion, of the swipe input).

In some examples, for instance with all (e.g., the entire portion) of the ECG 124 being moved to all (e.g., the entire portion) of the historical ECG 126 according to any of the above-mentioned techniques, all (e.g., the entire portion) of the GUI 120 is moved to all (e.g., the entire portion) of the pseudo-paper GUI 122. In those or other examples, the medical device 102, determining to move all (e.g., the entire portion) of the GUI 120 to all (e.g., the entire portion) of the pseudo-paper GUI 122, disregards any curvature and/or any arc associate with the swipe input.

In various implementations of the present disclosure, any of the characteristics associated with the swipe input identified as the touch input 132 is utilized to move to the pseudo-paper GUI 122 and/or the historical ECG 126, via the pan 134, in any way. For instance, any number and/or type of characteristics associated with swipe input, such as the length, the speed, the location (e.g., the start location, the end location, and/or one or more other locations), the curvature and/or the arc, and/or one or more other characteristics, are utilized to move to the pseudo-paper GUI 122 and/or the historical ECG 126. In some examples, any of the characteristics associated with swipe input is utilized to identify an amount of time (e.g., utilized to select the historical ECG 126), to identify whether the historical ECG 126 is dynamic or static, to identify whether or not to disregard the swipe input, to identify how much of the UI 104 is utilized for the pan 134, and/or to identify any other characteristics associated with the pan 134.

In various implementations, the UI 104 moves to the ECG 124 by the medical device 102 performing a snap operation (also simply referred to herein as "snap") 136. In some cases, the UI 104 moves from the historical ECG 126 to the ECG 124 by the medical device 102 performing the snap 136. For instance, the UI 104 moves from the historical ECG 126 to the ECG 124 via the snap 136 in response to receiving tap input (e.g., a tap) as the touch input 132. In such an instance or another instance, the tap input is received before or after the swipe input.

In various cases, the UI 104 moves to the ECG 124 via the snap 136 in response identifying, from among various possible types of input, that a type of touch input 132 is the tap input. In some examples, the medical device 102 identifies, selects, and/or presents the ECG 124 by identifying a current time, one or more other times of different types, or any combination thereof.

In some cases, the medical device 102 utilizes the snap 136 to snap (e.g., jump, skip, etc.) between the ECG 124 and the historical ECG 126. For example, the medical device 102 operates in the snapback mode to move, via the snap 136, between the historical ECG 126, the ECG 124, and/or one or more other ECGs.

In various cases, the medical device 102 operates in the snapback mode to quickly, easily, and without hesitation, move, via the snap 136, from the historical ECG 126 and to the ECG 124. For instance, the medical device 102 is quickly and easily controlled in response to receiving the snap 136 to promptly and immediately move the UI 104 to present the ECG 124. In various cases, by snapping and/or jumping to the ECG 124, instead of panning, the UI 104 prioritizes presenting important and life-saving ECG information that is being generated at current time.

The snap 136 is utilized to change the waveforms of the historical ECG 126 from being presented in the pseudo-paper format to being displayed to the electronic format. In some examples, the snap 136 is utilized to change from presenting, in the pseudo-paper format, all portions of the pseudo-paper GUI 122 to presenting, in the electronic format, all portions of the GUI 120. For instance, such as with respect to any of the techniques for moving between ECGs as discussed throughout this disclosure, all portions of the historical ECG 126 and/or the pseudo-paper GUI 122 are transitioned to all of the corresponding portions of the ECG 124 and/or the GUI 120 in unison (e.g., simultaneously). For instance, all of the waveforms of the historical ECG 126 are transitioned to all of the corresponding waveforms of the ECG 124 in unison (e.g., simultaneously, at the same speed/amount, in the same or different ways, or any combination thereof).

In alternative examples, the snap 136 is utilized to individually a change from presenting, in the pseudo-paper format, individual waveforms of the historical ECG 126 in the pseudo-paper GUI 122 to presenting, in the electronic format, individual waveforms of the ECG 124 of the GUI 120. For instance, the snap 136 is utilized to individually change from presenting, in the pseudo-paper format, a waveform of the historical ECG 126 in the pseudo-paper GUI 122 to presenting, in the electronic format, a waveform of the ECG 124 of the GUI 120. In various cases, the medical device 102 utilizes the snap 136 to change from presenting, in the pseudo-paper format, a particular waveform of the historical ECG 126 in the pseudo-paper GUI 122 to presenting, in the electronic format, a particular waveform of the ECG 124 of the GUI 120, in response to identifying that the touch input 132 includes a tap of the particular waveform. For example, the tap of the particular waveform includes a tap that overlaps with the waveform or, possible, a geometric shape (e.g., visible or not visible) outlining in the waveform.

In various implementations, the medical device 102 utilizes the device settings to control how the medical device 102 operates in response to receiving the touch input 132. For instance, any of the operations performed by the medical device 102 in response to identifying the characteristics of the touch input 132, and in response to identifying the settings, are adjustable. In some cases, the settings are adjustable (e.g., updateable) to enable any other operations to be utilized in response to identifying any other characteristics of the touch input 132, and in response to identifying the adjusted/updated settings. For instance, the medical device 102 is utilized to adjust any of the settings via a settings menu, any other type of screen and/or menu, or any combination thereof. In such an instance or another instance, the medical device 102 is utilized to adjust any of the settings in response to identifying one or more selections received via user input to the UI 104 (e.g., to the settings menu).

In various implementations of the present disclosure, the medical device 102 is configured to communicate with external devices. For example, the medical device 102 is configured to transmit and/or receive data with a wearable device 138 of the subject 128, a wearable device 140 of the user 130, a mobile device 142, a computing device 144, a display device 146, or any other type of electronic device configured to transmit and/or receive the data. The data is transmitted over one or more wireless communication links (e.g., a WI-FI® link, a WIGIG® link, a BLUETOOTH® link, a radio link, a near field communication (NFC) link, or the like), one or more wired communication links (e.g., an Ethernet link, an optical fiber link, or the like), or a combination thereof. In some cases, the data is transmitted over one or more communication networks, such as a local area network (LAN), a wide area network (WAN) (e.g., the Internet), a mobile core network (e.g., a 3rd Generation Partnership Project (3GPP) network), a radio access network (RAN), or any combination thereof. According to various examples, any of the wearable device 138 of the subject 128, the wearable device 140 of the user 130, the mobile device 142, the computing device 144, and the display device 146 includes a display configured to output the GUI 116 in any manner equivalent to the display of the medical device 102.

In some examples, the medical device 102 transmits data indicative of the GUI 120, the pseudo-paper GUI 122, or a combination thereof, to an external device (e.g., the wearable device 140 of the subject 128, the wearable device 140 of the user 130, the mobile device 142, the computing device 144, or the display device 146), thereby causing the external device to output any portion of the GUI 116 on its respective display. In some examples, any of the wearable device 140 of the subject 128, the wearable device 140 of the user 130, the mobile device 142, the computing device 144, and the display device 146 includes an input device configured to receive an input signal from the user 130 in any manner equivalent to the user input device 110 of the medical device 102. For example, the medical device 102 receives data indicative of the user input signal from an external device (e.g., the wearable device 140 of the subject 128, the wearable device 140 of the user 130, the mobile device 142, the computing device 144, or the display device 146), thereby enabling the user 130 to input signals to the medical device 102 via the external device.

The wearable device 140 of the subject 128 and the wearable device 140 of the user 130 are configured to receive and/or transmit data wirelessly with the medical device 102. In some examples, the wearable device 140 and/or the wearable device 140 includes a smartwatch, smart clothing, smart glasses, or any combination thereof. In various examples, the wearable device 140 is configured to be worn by the subject 128 and the wearable device 140 is configured to be worn by the user 130.

According to various implementations, the mobile device 142 is a user equipment (UE) configured to receive and/or transmit data wirelessly with the medical device 102. In some cases, the mobile device 142 includes a cellphone (e.g., a smartphone), a personal digital assistant (PDA), a tablet computer, or the like. In various examples, the mobile device 146 includes an internet of things (IoT) device.

In various examples, the computing device 144 includes any computing system configured to receive and/or transmit data with the medical device 102. In some cases, the computing device 144 communicates over one or more wired and/or wireless communication links. The computing device 144 includes, for example, a desktop computer, a laptop computer, a server computer, or any combination thereof.

The display device 146, for example, includes an external device configured to display the GUI 116 and/or other information output by the medical device 102. For example, the display device 146 includes a television (e.g., a smart TV), a monitor, a projector, or the like.

In some implementations, the medical device 102 can further be used to output at least a snapshot of the ECG 124. For instance, the medical device 102, in some implementations, may print or otherwise output a snapshot of the ECG 124 and/or the historical ECG 126 in response to detecting a user input signal. In particular cases, the medical device 102 prints the snapshot in response to detecting that a print button has been pressed by the user 130. In some examples, the medical device 102 saves the snapshot in memory in response to detecting that a save button has been pressed. In various cases, a log of data representing segment(s) of the ECG 124 and/or the historical ECG 126 is saved in memory.

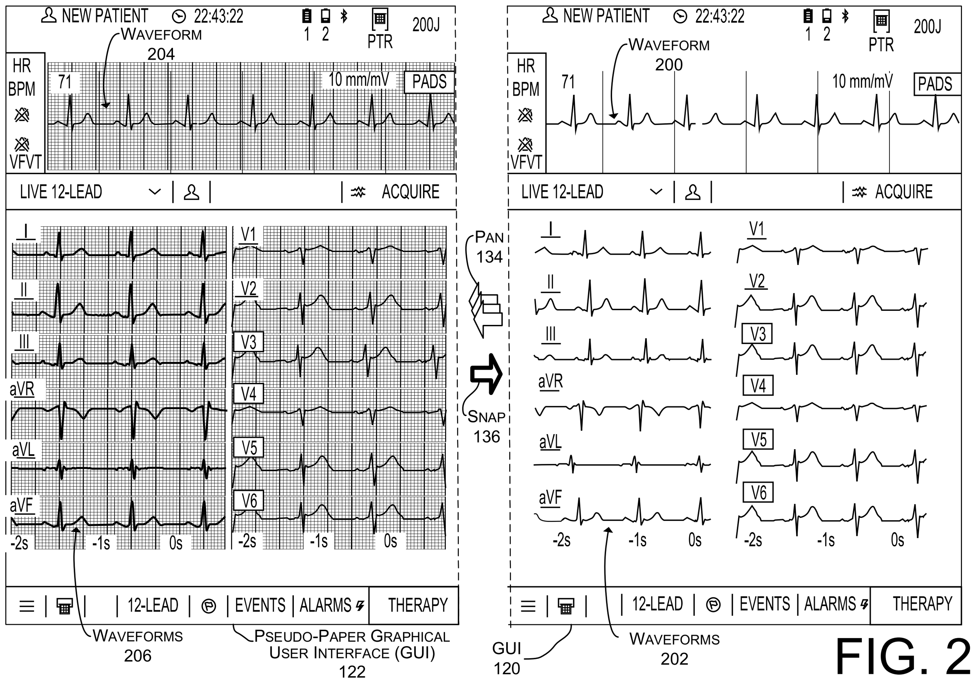

FIG. 2 illustrates a device that transitions between real-time and historical ECG modes. In various cases, the device (e.g., the medical device 102) operates in a real-time mode or an historical mode. In some examples, the medical device 102 outputs a waveform 200 in the real-time mode. In those or other examples, the medical device 102 operating in the real-time mode outputs waveforms 202 in an ECG (e.g., the ECG 124, as discussed above with reference to FIG. 1), via a GUI (e.g., the GUI 120, as discussed above with reference to FIG. 1). For instance, the waveforms 202 include, respectively, the waveforms of an ECG (e.g., the historical ECG 126, as discussed above with reference to FIG. 1), via a GUI (e.g., the pseudo-paper GUI 122, as discussed above with reference to FIG. 1).

In various cases, the waveform 200 is output in real-time, in an electronic format (e.g., instead of a pseudo-paper format). For instance, the waveform 200 is output in the GUI 120, along with the waveforms 202.

In some cases, the medical device 102 outputs, in the historical mode, a waveform 204. In those or other examples, the medical device 102 operating in the historical mode outputs waveforms 206 in a GUI (e.g., the pseudo-paper GUI 122, as discussed above with reference to FIG. 1). For instance, the waveforms 206 are included in an ECG (e.g., the historical ECG 126, as discussed above with reference to FIG. 1) being output, in the pseudo-paper GUI 122, by the medical device 102 operating in the historical mode.

In some cases, waveforms, including the waveform 200 and the waveforms 202, are moved to the waveform 204 and the waveforms 206, respectively. For example, the waveform 200 and the waveforms 202 are moved to the waveform 204 and the waveforms 206, respectively, via the pan 134. In various examples, the medical device 102 presenting the waveform 200 and the waveforms 202 moves to the waveform 204 and the waveforms 206, respectively, in response to receiving a swipe input as a touch input (e.g., the touch input 132).

In some cases, waveforms, including the waveform 204 and the waveforms 206, are moved to the waveform 200 and the waveforms 202, respectively. For example, the waveform 204 and the waveforms 206 are moved to the waveform 200 and the waveforms 202, respectively, via the snap 136. In various examples, the medical device 102 presenting the waveform 200 and the waveforms 202 moves to the waveform 204 and the waveforms 206, respectively, in response to receiving a tap input as the touch input 132.

In various examples, the medical device 102 identifies, via a touch (e.g., a third touch received) as input from the user to an input device (e.g., the UI 104), a command (e.g., a third command) to zoom in the snapshot of the ECG (e.g., a snapshot of any ECG) when the UI 104 is in any mode (e.g., a real-time mode, a pseudo-paper mode, etc.). For example, the third touch includes a tap, a double tap, or a pinch. In some cases, the medical device 102 identifies, via a touch (e.g., a fourth touch), received as input from the user to the input device of the medical device, a command (e.g., a fourth command) to pan in the snapshot of the ECG when the display is in the pseudo-paper mode. In some cases, the medical device 102 zooms in to a portion of a signal of the ECG in the snapshot of the ECG, the portion being identified by the third touch. pan to a different portion of the signal of the ECG, the different portion being identified by the fourth touch.

In various cases, the medical device 102, in response to identifying the first command, jumps the snapshot of the ECG linearly when the display is the backtrack mode (e.g., jumps from the ECG 124 to the historical ECG 126). In some examples, the medical device 102, in response to identifying the second command, jumps the snapshot of the ECG non-linearly when the display is in the snapback mode (e.g., jumps from the historical ECG 126 to the ECG 124). In various cases, the medical device 102, in response to identifying any of various commands, jumps linearly or non-linearly from the ECG 124 to the historical ECG 126, or vice versa.

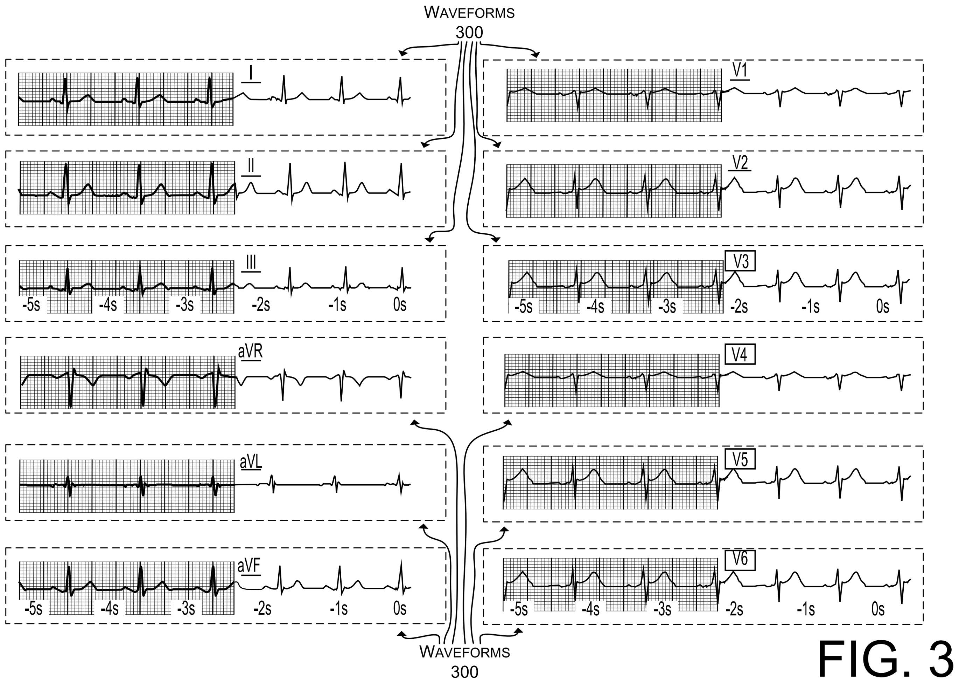

FIG. 3 illustrates a medical device that presents individual waveforms of an ECG in electronic and pseudo-paper formats. For instance, the waveforms include waveforms 300 (e.g., the waveforms 202, as discussed above with reference to FIG. 2) of an ECG (e.g., the historical ECG 126, as discussed above with reference to FIG. 1).

For example, a waveform 300 is presented by a medical device (e.g., the medical device 102) operating in any of various modes (e.g., e.g., an active mode, a real-time mode, a backtrack mode, a historical mode, a pseudo-paper mode, a snapback mode, one or more other modes of various types, or any combination thereof, as discussed above in FIGS 1 and 2). In some cases, the medical device 102 changes from the real-time mode to the backtrack mode with respect to the waveform 300. In various examples, changing from the real-time mode to the backtrack mode includes changing from presenting the waveform 300 in an electronic format, to presenting the waveform 300 in a pseudo-paper format.

In various cases, a real-time waveform of the waveform 300 transitions continually to a historical waveform of the waveform 300. For instance, the waveform 300 includes a portion (or "real-time portion") of the waveform 300 as the real-time waveform, and a portion (or " historical portion") of the waveform 300 as the historical waveform. The medical device 102 transitions smoothly from the real-time portion of the waveform 300 to the historical portion of the waveform 300. In some examples, the medical device 102 moves, by panning (e.g., via pan 134, as discussed above reference to FIG. 1) from the real-time portion of the waveform 300 to the historical portion of the waveform 300. In various cases, the medical device 102 moves, via the pan 134, from the real-time portion of the waveform 300 to the historical portion of the waveform 300, in response to receiving the swipe input as the touch input 132. In various cases, the medical device 102 moves, via the pan 134, from the real-time portion of the waveform 300 to the historical portion of the waveform 300, in response to receiving the swipe input in a location (e.g., any of the locations as discussed above with reference to FIG. 1) coinciding with (e.g., positioned within) a geometric shape (e.g., a rectangle, a circle, etc.) associated with the waveform 300. The geometric shape, for example, is a geometric shape (e.g., a visible or non-visible shape) in the UI 104 and surrounding (e.g., encompassing, enclosing, etc.) the waveform 300.

In some cases, the medical device 102 utilizes the swipe input to perform the pan 134 in response to characteristics of the swipe input. For instance, a touch (e.g., first touch) being received when the snapshot of the ECG is presented by the display in an active mode is identified as the swipe input by the medical device 102 that identifies a direction of the swipe is a predetermined direction (e.g., a left direction). In some cases, the medical device 102 determines to not perform the pan 134 in response to identifying a touch swipe in another direction (e.g., a right direction). In various examples, a swipe of a first type of swipe with an opposing direction, a faster speed, or a longer length than a second type of swipe is utilized to perform the pan 134. In some cases, a swipe of the second type of swipe is identified and utilized to refrain from performing the pan 134.

In various cases, the historical waveform of the waveform 300 transitions immediately, without presenting any intermediate portions (e.g., intermediate segments) of the waveform 300, from the historic waveform of the waveform 300 and to the real-time waveform of the waveform 300. For instance, the medical device 102 transitions jumps from the historical portion of the waveform 300 to the real-time portion of the waveform 300. In those or other examples, the medical device 102 moves, by snapping (e.g., via a snap 136, as discussed above reference to FIG. 1) from the real-time portion of the waveform 300 to the historical portion of the waveform 300. In various cases, the medical device 102 moves, via the snap 136, from the historical portion of the waveform 300 to the real-time portion of the waveform 300, in response to receiving the tap input as the touch input 132. For instance, the medical device 102 moves, via the snap 136, from the historical portion of the waveform 300 to the real-time portion of the waveform 300, in response to receiving the tap input in a location coinciding with (e.g., positioned within) a geometric shape (e.g., a rectangle) associated with the waveform 300. In various cases, as time passes, the intermediate portions of the waveform 300 increase in time as the real-time portion of the waveform 300 becomes further (in time) from the historic waveform.

In some examples, the medical device 102 moves from the GUI 120 and to the pseudo-paper GUI 122 by identifying and/or generating a command in response to the swipe input. For instance, the command in response to the swipe input is utilized to initiate the pan 134. In those or other examples, the medical device 102 moves from the pseudo-paper GUI 122 and to the GUI 120 by identifying and/or generating a command in response to the tap input. For instance, the command in response to the tap input is utilized to initiate the snap 136.

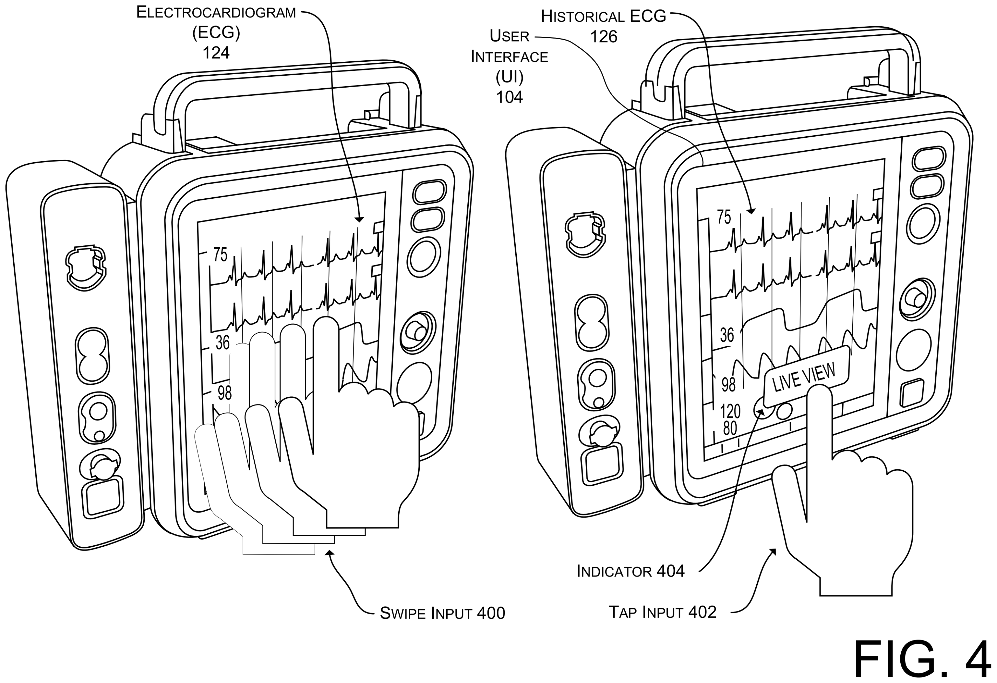

FIG. 4 illustrates an example environment in which a UI of a device receives user swipe or tap input to request the UI to pan or snap between a real-time and pseudo-paper electronic electrocardiogram. In some examples, the device (e.g., the medical device 102) includes the UI (e.g., the UI 104, as discussed above with reference to FIG. 1) that graphically outputs physiological parameters of a subject (e.g., the subject 128, as discussed above with reference to FIG. 1). For instance, the physiological parameters include an ECG (e.g., the ECG 124, as discussed above with reference to FIG. 1). In various cases, the physiological parameters include a 12-lead ECG as the ECG 124, with one or more waveforms (e.g., a waveform 200, as discussed above with reference to FIG. 2). In some examples, 12 waveforms corresponding to 12-leads of the ECG 124 are presented by the UI 104.

In various examples, the medical device 102 presents, in a GUI (e.g., the GUI 120), the ECG 124 in response to being activated. For example, the medical device 102 presents the ECG 124 in the GUI 120 in response to being initially activated, turned on, powered on, booted up, prior to any settings (e.g., the settings, as discussed above with reference to FIG. 1) being set and/or after any of the settings being set. For instance, the medical device 102 presents the ECG 124 in the activate mode, which includes the real-time mode. In those or other instances, the medical device 102 presents the ECG 124 in the activate mode (e.g., the real-time mode), in response to identifying the physiological parameters of the subject 128. In those or other instances. In some cases, prior to receiving the swipe input 400, the medical device 102 presents, via the GUI 120, the waveform 200 and/or the waveforms 202, as discussed above with reference to FIG. 2.

In various cases, the medical device 102 changes from a real-time mode to a backtrack mode in response to receiving a swipe input 400, as a touch input (e.g., the touch input 132). In some examples, the medical device 102 receiving the swipe input 400 (e.g., any swipe input, as discussed above with reference to FIG. 1) as the subsequent touch input (e.g., another touch input 132) includes the medical device 102 receiving a swipe via the UI 104 and from a user (e.g., the user 130). In some cases, the medical device 102 receiving the swipe input 400 moves to the backtrack mode and then the historical mode. For instance, the medical device 102 receiving the swipe input 400, and moving to the backtrack mode and then the historical mode, presents the historical ECG 126. In some cases, the medical device presents the ECG 124 at a time (e.g., a time t1) and presents the historical ECG 126 at a time (e.g., a time t2) subsequent to the time t1.

In some examples, the medical device 102 sets a tag, in response to receiving a swipe input 400. For instance, the tag (e.g., a snapback tag) is set and utilized to enable (e.g., trigger) the medical device 102 to automatically determine a response (e.g., a snapback response) to move to a snapback mode in response to receiving a touch input (e.g., a subsequent touch input, such as a tap input). In some cases, the medical device 102 receives the tap input, which includes a tap input 402, as the subsequent touch input (e.g., another touch input 132). For example, the medical device 102 receiving the tap input 402 includes the medical device 102 receiving a tap via the UI 104 and from the user 130. In various instances, the snapback tag is utilized to enable (e.g., trigger) the medical device 102 to automatically move to the snapback mode in response to receiving any type of input after the snapback tag is set. For instance, the medical device 102 moves to the snapback mode, and then the real-time mode. In various examples, the medical device 102 receiving the tap input 402, and moving to the backtrack mode and then the historical mode, presents the historical ECG 126. In some cases, the medical device 102 presents historical ECG 126 at the time t2 and presents the ECG 124 at a time (e.g., a time t3) subsequent to the time t2. For instance, the medical device 102, operating in the historical mode, presents historical ECG 126 at the time t2. In such an instance or another instance, the medical device 102, operating in the real-time mode, presents the ECG 124 at the time t3.

In various examples, the medical device 102 presents, in a pseudo-paper GUI (e.g., the pseudo-paper GUI 122), the historical ECG 126 in response to receiving the swipe input 400. In some cases, the pseudo-paper GUI 122 is utilized to present the waveform 204 and/or the waveforms 206.

In various cases, the medical device 102 presents, via the UI 104, an indicator (e.g., a button), in response to receiving the swipe input 400. For instance, the medical device 102, entering the historical mode (e.g., during and/or after entering the backtrack mode) presents the indicator, such as an indicator 404. In some examples, the indicator 404 is presented by the UI 104 superimposing the indicator 404 on the historical ECG 126. In those or other examples, the indicator 404 is presented by the UI 104 on any portion of the UI 104, such as in any portion of the pseudo-paper GUI 122. In some examples, the indicator 404 is presented below, to a side, above, etc., of the historical ECG 126, the waveform 204, any of the waveforms 206, and/or any other portion of the UI 104.

In various cases, a single indicator (e.g., the indicator 404) is presented, and connected to the historical ECG 126 (e.g., all of the historical ECG 126). For instance, the single indicator is connected to the waveform 204 and/or the waveforms 206. In some examples, the indicator being selected by the tap input 402 is utilized to move a mode associated with the historical ECG 126 to a real-time mode (e.g., the historical ECG 126 is moved to the ECG 124). In those or other examples, the indicator being selected by the tap input 402 is utilized to move a mode associated with the waveform 204 and/or the waveforms 206 to a real-time mode (e.g., the waveform 204 and/or the waveforms 206 are moved to the waveform 200 and/or the waveforms 202, respectively). For example, if one or more of the waveforms 202 are moved to one or more corresponding waveforms 206, all of the one or more waveforms 206 are moved back to the one or more waveforms 202 after the indicator 404 (e.g., for the historical ECG 126) is selected by the tap input 402. In some cases, any tap input 402 (e.g., associated with, and/or overlapping, the historical ECG 126; and/or within a shape outlining the historical ECG 126, the waveform 200, and/or the waveforms 202) is utilized in a similar way as the indicator 404 (e.g., for all of the historical ECG 126), such as based on a default setting of the medical device 102 and/or based on updates to the device settings.

In some examples, individual indicators (e.g., an indicator 404) is presented, and connected to the waveform 204 or a corresponding one of the waveforms 206. In various cases, the indicator being selected by the tap input 402 is utilized to move a mode associated with the waveform 204 or the waveform 206 to a real-time mode (e.g., the waveform 204 is moved to the waveform 200) (e.g., the waveform 206 is moved to a corresponding waveform 202). For example, if the waveform 200 or a single waveform of the waveforms 202 is moved to a corresponding waveform 206, the waveform 204 or the waveform 206 is moved back to the waveform 200 or the waveform 202, respectively, after the indicator 404 (e.g., for the waveform 204 or the individual waveform 206) is selected by the tap input 402. In some cases, any tap input 402 (e.g., associated with, and/or overlapping, the waveform 204 or the individual waveform 206; and/or within a shape outlining the waveform 204 or the individual waveform 206) is utilized in a similar way as the indicator 404 (e.g., for the waveform 204 or the individual waveform 206), such as based on a default setting of the medical device 102 and/or based on updates to the device settings.

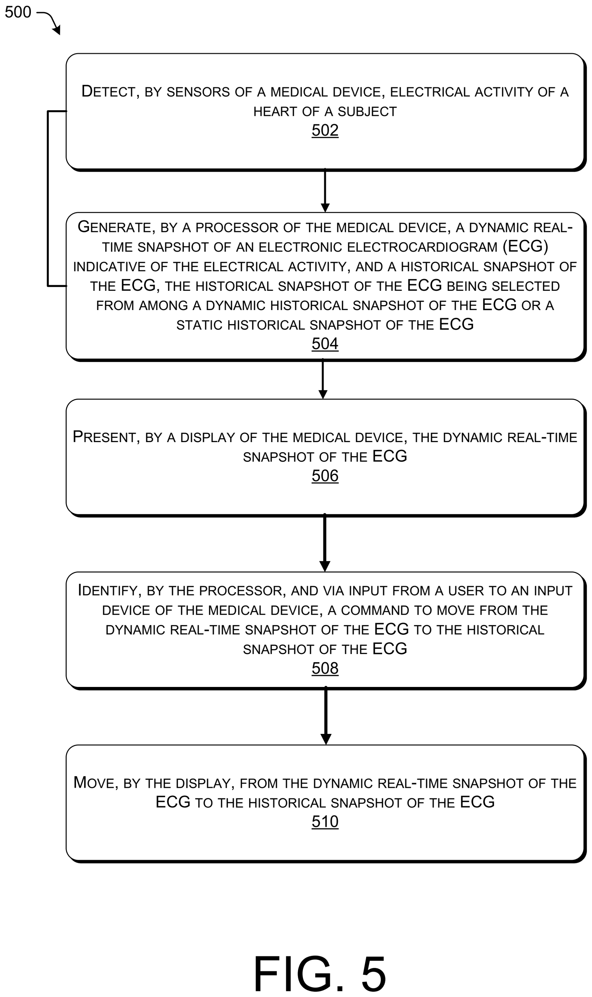

FIG. 5 illustrates an example process 500 for presenting a dynamic real-time snapshot of an ECG and moving from the dynamic real-time snapshot of the ECG to the historical snapshot of the ECG. In some examples, a device for which the dynamic real-time snapshot of the ECG is presented and moved to the historical snapshot of the ECG includes the medical device 102, as discussed above with reference to FIG. 1.

At 502, the process 500 includes detecting, by sensors of a medical device, electrical activity of a heart of a subject. For example, the medical device (e.g., the medical device 102) receives the activity of the heart of the subject, such as a subject 128. The medical device 102 identifies physiological parameters, including an ECG, such as the ECG 124 and the historical ECG 126.

At 504, the process 500 includes generating, by a processor of the medical device, a dynamic real-time snapshot of an electronic ECG indicative of the electrical activity, and a historical snapshot of the ECG, the historical snapshot of the ECG being selected from among a dynamic historical snapshot of the ECG or a static historical snapshot of the ECG. In some examples, the medical device 102 generates the dynamic real-time snapshot of an electronic ECG, such as the ECG 124, and the historical snapshot of the ECG, such as the historical ECG 126. For instance, the historical snapshot is selected from among the dynamic historical snapshot of the ECG or the static historical snapshot of the ECG.

At 506, the process 500 includes presenting, by a display of the medical device, the dynamic real-time snapshot of the ECG. For example, the medical device 102 presents the dynamic real-time snapshot, including the ECG 124.

At 508, the process 500 includes identifying, by the processor, and via input from a user to an input device of the medical device, a command to move from the dynamic real-time snapshot of the ECG to the historical snapshot of the ECG. For examples, the medical device 102 identifies to move from the ECG 124 to the historical ECG 126, and moves from the dynamic real-time snapshot of the ECG 124 to the historical snapshot of the historical ECG 126.

At 510, the process 500 includes moving, by the display, from the dynamic real-time snapshot of the ECG to the historical snapshot of the ECG. For example, the display, such as the UI 104, moves from the ECG 124 and to the historical ECG 126. In some cases, all 12 waveforms of the ECG 124 are moved to all 12 waveforms of the historical ECG 126. For instance, all of the waveforms 202 are moved to all of the waveforms 206, simultaneously. In such an instance or another instance, the waveforms 202 are panned, in unison, to all the waveforms 206. Alternatively or additionally, the waveform 200 is moved (e.g., panned, in unison with the waveforms 202) to the waveform 204.

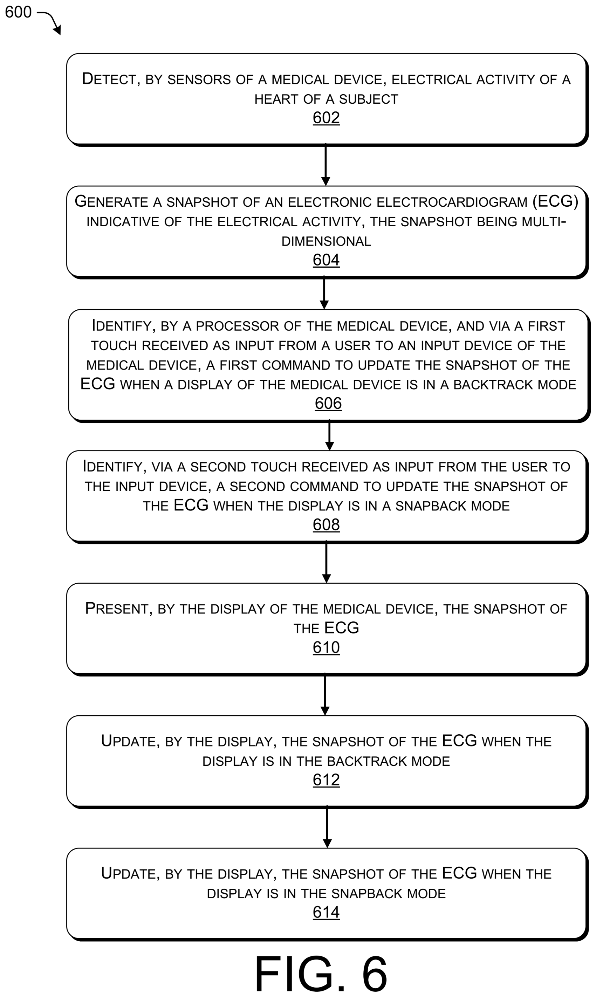

FIG. 6 illustrates an example process 600 for presenting a snapshot of an ECG, updating the snapshot when the display is in a backtrack mode, and updating the snapshot when the display is in a snapback mode. In some examples, a device that updates the snapshot when the display is in the backtrack mode, and updates the snapshot when the display is in the snapback mode includes the medical device 102, as discussed above with reference to FIG. 1.

At 602, the process 600 includes detecting, by sensors of a medical device, electrical activity of a heart of a subject. For instance, the medical device 102 is utilized to present, via a UI 104, an ECG 124 and one or more historical ECG, such as a historical ECG 126. In some examples, the ECG 124 includes waveforms 202; and the historical ECG 126 includes waveforms 206.

At 604, the process 600 includes generating a snapshot of an electronic ECG indicative of the electrical activity, the snapshot being multi-dimensional. For example, a dimension of the snapshot includes the ECG captured a specific point in time, a subsequent dimension of the snapshot includes the ECG captured a subsequent point in time, and so on. In various cases, the dimensions of the snapshot include one or more temporal dimensions.

At 606, the process 600 includes identifying, by a processor of the medical device, and via a first touch received as input from a user to an input device of the medical device, a first command to update the snapshot of the ECG when a display of the medical device is in a backtrack mode. In some examples, the medical device 102 receives the first touch, to the UI 104, as the touch input 132. In those or other examples, the medical device 102 generates and/or identifies the first command. For instance, the first command us utilized to update the snapshot of the ECG (e.g., the ECG 124), when the display (e.g., the UI 104) is in the backtrack mode. In some cases, the updating of the snapshot occurs when the medical device 102 operates in the backtrack mode. In various examples, completion of the backtrack mode is utilized to present the historical ECG 126, in the historical mode.

At 608, the process 600 includes identifying, via a second touch received as input from the user to the input device, a second command to update the snapshot of the ECG when the display is in a snapback mode. In some examples, the medical device 102 receives the second touch, to the UI 104. For instance, the medical device 102 generates the second command and updates the snapshot of the ECG (e.g., the historical ECG 126).

At 610, the process 600 includes presenting, by the display of the medical device, the snapshot of the ECG. In some examples, the medical device 102 presents the ECG 124 in the real-time mode, and subsequently presents the historical ECG 126 in the historical mode.

At 612, the process 600 includes updating, by the display, the snapshot of the ECG when the display is in the backtrack mode. For instance, the UI 104 is utilized to update the ECG 124 to be the historical ECG 126 in the backtrack mode. In some examples, the UI 104 moves to the historical ECG 126 by panning to the historical ECG 126.

At 614, the process 600 includes updating, by the display, the snapshot of the ECG when the display is in the snapback mode. For instance, the UI 104 is utilized to update the historical ECG 126 to be the ECG 124 in the snapback mode. In some examples, the UI 104 moves to the ECG 124 by jumping to the ECG 124.

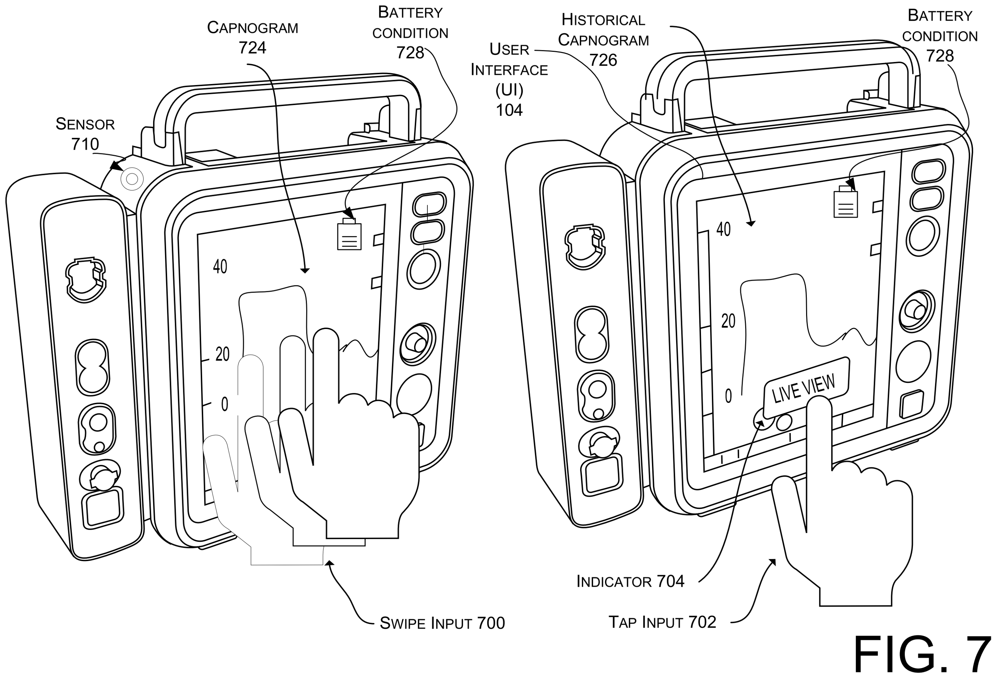

FIG. 7 illustrates an example environment in which a UI of a device receives user swipe or tap input to request the UI to pan or snap between a real-time and pseudo-paper electronic display. In some examples, the device (e.g., the medical device 102) includes the UI (e.g., the UI 104, as discussed above with reference to FIG. 1) that graphically outputs physiological parameters of a subject (e.g., the subject 128, as discussed above with reference to FIG. 1). For instance, the physiological parameters may include end tidal $CO_2$ as shown in a capnogram, $SPO_2$ plethysmography, blood pressure, and patient temperature. In other examples, the UI graphically or digitally display information relating to the functioning of the device such as the battery condition, temperature of a device, or connectivity of a device. In other examples, the UI 104 may graphically or digitally display information relating to the environment in which the device is located such as the temperature, humidity, motion, or water exposure.

For example, the housing of the medical device may include a sensor 710. While the term sensor is used broadly, the sensor may also be an indicator, tag, or hologram. The sensor may detect chemical, enzymatic, mechanical, electrochemical, or microbiological reactions. In some aspects, the sensor is a diode. In other aspects, the sensor is a thermocouple. In other aspects, the sensor may be a position sensor, an RFID chip, an accelerometer, piezoelectric crystals, strain gauges, pressure sensor, force sensor, particle sensor, radiation sensor, electrical sensor, colorimetric sensor, thermochromic ink, or gyroscope. Such a sensor as exterior sensor 710 may be used to measure the environmental conditions to which the medical device has been exposed and display the information on the UI 104. Such information may be displayed in a graph as individual points or as a series of waveforms. Battery condition 728 is an exemplary icon that may be used to display real-time and historical information about the functioning of the device. For example, battery condition 728 could be displayed graphically as a function of time depicting the speed at which the battery drains.

In various examples, the medical device 102 presents, in a GUI (e.g., the GUI 120), the capnogram 724 in response to being activated. For example, the medical device 102 presents the capnogram 724 in the GUI 120 in response to being initially activated, turned on, powered on, booted up, prior to any settings (e.g., the settings, as discussed above with reference to FIG. 1) being set and/or after any of the settings being set. For instance, the medical device 102 presents the capnogram 724 in the activate mode, which includes the real-time mode. In those or other instances, the medical device 102 presents the capnogram 724 in the activate mode (e.g., the real-time mode), in response to identifying the physiological parameters of the subject 128.

In various cases, the medical device 102 changes from a real-time mode to a backtrack mode in response to receiving a swipe input 700, as a touch input (e.g., the touch input 132). In some examples, the medical device 102 receiving the swipe input 700 (e.g., any swipe input, as discussed above with reference to FIG. 1) as the subsequent touch input (e.g., another touch input 132) includes the medical device 102 receiving a swipe via the UI 104 and from a user (e.g., the user 130). In some cases, the medical device 102 receiving the swipe input 700 moves to the backtrack mode and then the historical mode. For instance, the medical device 102 receiving the swipe input 700, and moving to the backtrack mode and then the historical mode, presents the historical capnogram 726. In some cases, the medical device presents the capnogram 724 at a time (e.g., a time t1) and presents the historical capnogram 726 at a time (e.g., a time t2) subsequent to the time t1.

In some examples, the medical device 102 sets a tag, in response to receiving a swipe input 700. For instance, the tag (e.g., a snapback tag) is set and utilized to enable (e.g., trigger) the medical device 102 to automatically determine a response (e.g., a snapback response) to move to a snapback mode in response to receiving a touch input (e.g., a subsequent touch input, such as a tap input). In some cases, the medical device 102 receives the tap input, which includes a tap input 702, as the subsequent touch input (e.g., another touch input 132). For example, the medical device 102 receiving the tap input 702 includes the medical device 102 receiving a tap via the UI 104 and from the user 130. In various instances, the snapback tag is utilized to enable (e.g., trigger) the medical device 102 to automatically move to the snapback mode in response to receiving any type of input after the snapback tag is set. For instance, the medical device 102 moves to the snapback mode, and then the real-time mode. In various examples, the medical device 102 receiving the tap input 702, and moving to the backtrack mode and then the historical mode, presents the historical capnogram 726. In some cases, the medical device 102 presents historical capnogram 726 at the time t2 and presents the historical capnogram 726 at a time (e.g., a time t3) subsequent to the time t2. For instance, the medical device 102, operating in the historical mode, presents historical capnogram 726 at the time t2. In such an instance or another instance, the medical device 102, operating in the real-time mode, presents the capnograph at the time t3.

In various examples, the medical device 102 presents, in a pseudo-paper GUI (e.g., the pseudo-paper GUI 122), the historical capnogram 726 in response to receiving the swipe input 700. In some cases, the pseudo-paper GUI 122 is utilized to present waveforms of the information.

In various cases, the medical device 102 presents, via the UI 104, an indicator (e.g., a button), in response to receiving the swipe input 700. For instance, the medical device 102, entering the historical mode (e.g., during and/or after entering the backtrack mode) presents the indicator, such as an indicator 704. In some examples, the indicator 704 is presented by the UI 104 superimposing the indicator 704 on the historical capnogram 726. In those or other examples, the indicator 704 is presented by the UI 104 on any portion of the UI 104, such as in any portion of the pseudo-paper GUI 122. In some examples, the indicator 704 is presented below, to a side, above, etc., of the historical capnogram 726, the waveform 204, any of the waveforms 206, and/or any other portion of the UI 104.

In various cases, a single indicator (e.g., the indicator 704) is presented, and connected to the historical capnogram 726 (e.g., all of the historical capnogram 726). For instance, the single indicator is connected to the waveform 204 and/or the waveforms 206. In some examples, the indicator being selected by the tap input 702 is utilized to move a mode associated with the historical capnogram 726 to a real-time mode (e.g., the historical capnogram 726 is moved to the capnogram 724). In those or other examples, the indicator being selected by the tap input 702 is utilized to move a mode associated with a waveform to a real-time mode. For example, if one or more of the waveforms A are moved to one or more corresponding waveforms B, all of the one or more waveforms B are moved back to the one or more waveforms after the indicator 704 (e.g., for the historical capnogram 726) is selected by the tap input 702 (an example of this is shown with reference to the ECG of FIG. 2 with waveform 204 an example of a waveform A and waveform 206 an example of a waveform B). In some cases, any tap input 702 (e.g., associated with, and/or overlapping, the historical capnogram 726; and/or within a shape outlining the historical capnogram 726) is utilized in a similar way as the indicator 704 (e.g., for all of the historical capnogram 726), such as based on a default setting of the medical device 102 and/or based on updates to the device settings. In some examples, individual indicators (e.g., an indicator 704) is presented, and connected to the waveform A or a corresponding one of the waveforms B In various cases, the indicator being selected by the tap input 702 is utilized to move a mode associated with the waveform A or the waveform B to a real-time mode (e.g., the waveform A is moved to the waveform B) (e.g., the waveform B is moved to a corresponding waveform A).

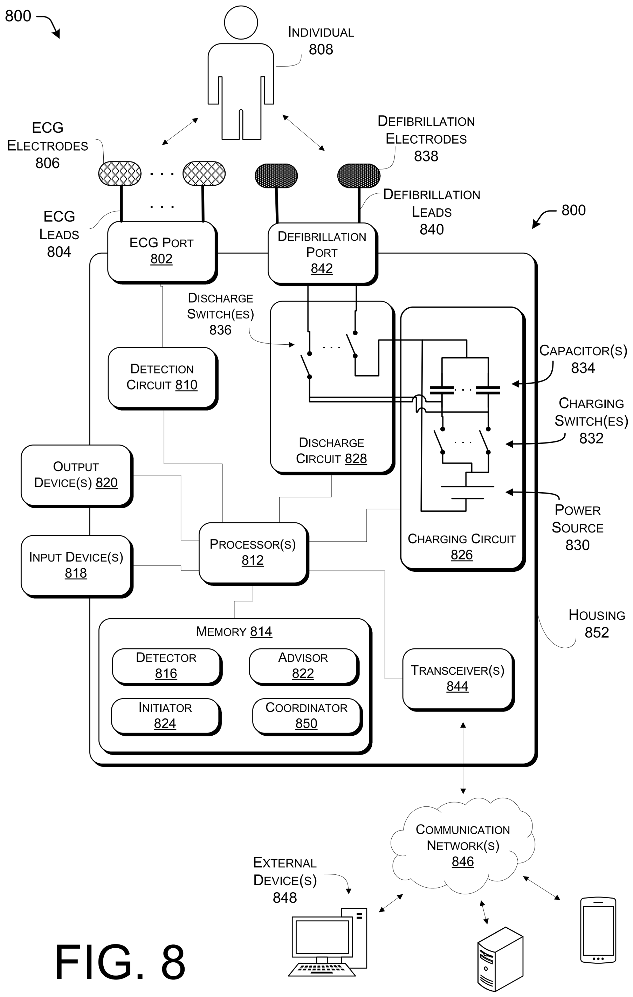

FIG. 8 illustrates an example of an external defibrillator 800 configured to perform various functions described herein. The external defibrillator 800 includes an ECG port 802 connected to multiple ECG leads 804. For instance, the external defibrillator 800 is utilized to implement the medical device 102. In some cases, the ECG leads 804 are removeable from the ECG port 802. For instance, the ECG leads 804 are plugged into the ECG port 802. The ECG leads 804 are connected to ECG electrodes 806, respectively. In various implementations, the ECG electrodes 806 are disposed on different locations on an individual 808. A detection circuit 810 is configured to detect relative voltages between the ECG electrodes 806. These voltages are indicative of the electrical activity of the heart of the individual 808.

In various implementations, the ECG electrodes 806 are in contact with the different locations on the skin of the individual 808. In some examples, a first one of the ECG electrodes 806 is placed on the skin between the heart and right arm of the individual 808, a second one of the ECG electrodes 806 is placed on the skin between the heart and left arm of the individual 808, and a third one of the ECG electrodes 806 is placed on the skin between the heart and a leg (either the left leg or the right leg) of the individual 808. In these examples, the detection circuit 810 is configured to measure the relative voltages between the first, second, and third ECG electrodes 806. Respective pairings of the ECG electrodes 806 are referred to as "leads," and the voltages between the pairs of ECG electrodes 806 are known as "lead voltages." In some examples, more than three ECG electrodes 806 are included, such that 6-lead or 12-lead ECG signals are detected by the detection circuit 810.

The detection circuit 810 includes at least one analog circuit, at least one digital circuit, or a combination thereof. The detection circuit 810 receives the analog electrical signals from the ECG electrodes 806, via the ECG port 802 and the ECG leads 804. In some cases, the detection circuit 810 includes one or more analog filters configured to filter noise and/or artifact from the electrical signals. The detection circuit 810 includes an analog-to-digital (ADC) in various examples. The detection circuit 810 generates a digital signal indicative of the analog electrical signals from the ECG electrodes 806. This digital signal can be referred to as an "ECG signal" or an "ECG."

In some cases, the detection circuit 810 further detects an electrical impedance between at least one pair of the ECG electrodes 806. For example, the detection circuit 810 includes, or otherwise controls, a power source that applies a known voltage (or current) across a pair of the ECG electrodes 806 and detects a resultant current (or voltage) between the pair of the ECG electrodes 806. The impedance is generated based on the applied signal (voltage or current) and the resultant signal (current or voltage). In various cases, the impedance corresponds to respiration of the individual 808, chest compressions performed on the individual 808, and other physiological states of the individual 808. In various examples, the detection circuit 810 includes one or more analog filters configured to filter noise and/or artifact from the resultant signal. The detection circuit 810 generates a digital signal indicative of the impedance using an ADC. This digital signal can be referred to as an "impedance signal" or an "impedance."

The detection circuit 810 provides the ECG signal and/or the impedance signal one or more processors 812 in the external defibrillator 800. In some implementations, the processor(s) 812 includes a central processing unit (CPU), a graphics processing unit (GPU), both CPU and GPU, or other processing unit or component known in the art.

The processor(s) 812 is operably connected to memory 814. In various implementations, the memory 814 is volatile (such as random access memory (RAM)), nonvolatile (such as read only memory (ROM), flash memory, etc.) or some combination of the two. The memory 814 stores instructions that, when executed by the processor(s) 812, causes the processor(s) 812 to perform various operations. In various examples, the memory 814 stores methods, threads, processes, applications, objects, modules, any other sort of executable instruction, or a combination thereof. In some cases, the memory 814 stores files, databases, or a combination thereof. In some examples, the memory 814 includes, but is not limited to, RAM, ROM, electrically erasable programmable read-only memory (EEPROM), flash memory, or any other memory technology. In some examples, the memory 814 includes one or more of CD-ROMs, digital versatile discs (DVDs), content-addressable memory (CAM), or other optical storage, magnetic cassettes, magnetic tape, magnetic disk storage or other magnetic storage devices, or any other medium which can be used to store the desired information and which can be accessed by the processor(s) 812 and/or the external defibrillator 800. In some cases, the memory 814 at least temporarily stores the ECG signal and/or the impedance signal.

In various examples, the memory 814 includes a detector 816, which causes the processor(s) 812 to determine, based on the ECG signal and/or the impedance signal, whether the individual 808 is exhibiting a particular heart rhythm. For instance, the processor(s) 812 determines whether the individual 808 is experiencing a shockable rhythm that is treatable by defibrillation. Examples of shockable rhythms include ventricular fibrillation (VF) and ventricular tachycardia (V-Tach). In some examples, the processor(s) 812 determines whether any of a variety of different rhythms (e.g., asystole, sinus rhythm, atrial fibrillation (AF), etc.) are present in the ECG signal.

The processor(s) 812 is operably connected to one or more input devices 818 and one or more output devices 820. Collectively, the input device(s) 818 and the output device(s) 820 function as an interface between a user and the defibrillator 800. The input device(s) 818 is configured to receive an input from a user and includes at least one of a keypad, a cursor control, a touch-sensitive display, a voice input device (e.g., a microphone), a haptic feedback device (e.g., a gyroscope), or any combination thereof. The output device(s) 820 includes at least one of a display, a speaker, a haptic output device, a printer, or any combination thereof. In various examples, the processor(s) 812 causes a display among the input device(s) 818 to visually output a waveform of the ECG signal and/or the impedance signal. In some implementations, the input device(s) 818 includes one or more touch sensors, the output device(s) 820 includes a display screen, and the touch sensor(s) are integrated with the display screen. Thus, in some cases, the external defibrillator 800 includes a touchscreen configured to receive user input signal(s) and visually output physiological parameters, such as the ECG signal and/or the impedance signal.

In some examples, the memory 814 includes an advisor 822, which, when executed by the processor(s) 812, causes the processor(s) 812 to generate advice and/or control the output device(s) 820 to output the advice to a user (e.g., a rescuer). In some examples, the processor(s) 812 provides, or causes the output device(s) 820 to provide, an instruction to perform CPR on the individual 808. In some cases, the processor(s) 812 evaluates, based on the ECG signal, the impedance signal, or other physiological parameters, CPR being performed on the individual 808 and causes the output device(s) 820 to provide feedback about the CPR in the instruction. According to some examples, the processor(s) 812, upon identifying that a shockable rhythm is present in the ECG signal, causes the output device(s) 820 to output an instruction and/or recommendation to administer a defibrillation shock to the individual 808.

The memory 814 also includes an initiator 824 which, when executed by the processor(s) 812, causes the processor(s) 812 to control other elements of the external defibrillator 800 in order to administer a defibrillation shock to the individual 808. In some examples, the processor(s) 812 executing the initiator 824 selectively causes the administration of the defibrillation shock based on determining that the individual 808 is exhibiting the shockable rhythm and/or based on an input from a user (received, e.g., by the input device(s) 818. In some cases, the processor(s) 812 causes the defibrillation shock to be output at a particular time, which is determined by the processor(s) 812 based on the ECG signal and/or the impedance signal.

The processor(s) 812 is operably connected to a charging circuit 826 and a discharge circuit 828. In various implementations, the charging circuit 826 includes a power source 830, one or more charging switches 832, and one or more capacitors 834. The power source 830 includes, for instance, a battery. The processor(s) 812 initiates a defibrillation shock by causing the power source 830 to charge at least one capacitor among the capacitor(s) 834. For example, the processor(s) 812 activates at least one of the charging switch(es) 832 in the charging circuit 826 to complete a first circuit connecting the power source 830 and the capacitor to be charged. Then, the processor(s) 812 causes the discharge circuit 828 to discharge energy stored in the charged capacitor across a pair of defibrillation electrodes 838, which are in contact with the individual 808. For example, the processor(s) 812 deactivates the charging switch(es) 832 completing the first circuit between the capacitor(s) 834 and the power source 830, and activates one or more discharge switches 836 completing a second circuit connecting the charged capacitor 834 and at least a portion of the individual 808 disposed between defibrillation electrodes 838.

The energy is discharged from the defibrillation electrodes 838 in the form of a defibrillation shock. For example, the defibrillation electrodes 838 are connected to the skin of the individual 808 and located at positions on different sides of the heart of the individual 808, such that the defibrillation shock is applied across the heart of the individual 808. The defibrillation shock, in various examples, depolarizes a significant number of heart cells in a short amount of time. The defibrillation shock, for example, interrupts the propagation of the shockable rhythm (e.g., VF or V-Tach) through the heart. In some examples, the defibrillation shock is 200 J or greater with a duration of about 0.016 seconds. In some cases, the defibrillation shock has a multiphasic (e.g., biphasic) waveform. The discharge switch(es) 836 are controlled by the processor(s) 812, for example. In various implementations, the defibrillation electrodes 838 are connected to defibrillation leads 840. The defibrillation leads 840 are connected to a defibrillation port 842, in implementations. According to various examples, the defibrillation leads 840 are removable from the defibrillation port 842. For example, the defibrillation leads 840 are plugged into the defibrillation port 842.

In various implementations, the processor(s) 812 is operably connected to one or more transceivers 844 that transmit and/or receive data over one or more communication networks 846. For example, the transceiver(s) 844 includes a network interface card (NIC), a network adapter, a local area network (LAN) adapter, or a physical, virtual, or logical address to connect to the various external devices and/or systems. In various examples, the transceiver(s) 844 includes any sort of wireless transceivers capable of engaging in wireless communication (e.g., radio frequency (RF) communication). For example, the communication network(s) 846 includes one or more wireless networks that include a 3rd Generation Partnership Project (3GPP) network, such as a Long Term Evolution (LTE) radio access network (RAN) (e.g., over one or more LTE bands), a New Radio (NR) RAN (e.g., over one or more NR bands), or a combination thereof. In some cases, the transceiver(s) 844 includes other wireless modems, such as a modem for engaging in WI-FI®, WIGIG®, WIMAX®, BLUETOOTH®, NFC, radio frequency identification (RFID), or infrared communication over the communication network(s) 846.

The defibrillator 800 is configured to transmit and/or receive data (e.g., ECG data, impedance data, data indicative of one or more detected heart rhythms of the individual 808, data indicative of one or more defibrillation shocks administered to the individual 808, etc.) with one or more external devices 848 via the communication network(s) 846. The external devices 848 include, for instance, mobile devices (e.g., mobile phones, smart watches, etc.), Internet of Things (IoT) devices, medical devices, computers (e.g., laptop devices, servers, etc.), or any other type of computing device configured to communicate over the communication network(s) 846. In some examples, the external device(s) 848 is located remotely from the defibrillator 800, such as at a remote clinical environment (e.g., a hospital). According to various implementations, the processor(s) 812 causes the transceiver(s) 844 to transmit data to the external device(s) 848. In some cases, the transceiver(s) 844 receives data from the external device(s) 848 and the transceiver(s) 844 provide the received data to the processor(s) 812 for further analysis.

In some cases, the external device(s) 848 include one or more medical devices. According to various implementations, the memory 814 further includes a coordinator 850 which, when executed by the processor(s) 812, causes the processor(s) 812 to coordinate with the external device(s) 848, such as by administering therapy (e.g., defibrillation, pacing, etc.) to a subject based on communication between the defibrillator 800 and the external device(s) 848, as described herein. In some implementations, the processor(s) 812, when executing the coordinator 850, receives data from the external device(s) 848, analyzes the data to determine a control parameter(s), and administers therapy (e.g., defibrillation, pacing, etc.) in accordance with the control parameter(s), as described herein. In some implementations, the processor(s) 812, when executing the coordinator 850, determines a parameter associated with the therapy being administered by the defibrillator 800, such as a physiological parameter of the individual 808, determines, by analyzing the parameter, a control parameter for controlling administration of therapy to the individual 808 by the external device(s) 848, and sends data (e.g., via the transceiver(s) 844) to the external device(s) 848, the data representing the control parameter. In general, the coordinator 850, when executed by the processor(s) 812, may cause the processor(s) 812 to perform any of the processes 500 and 600 described herein.

In various implementations, the external defibrillator 800 also includes a housing 852 that at least partially encloses other elements of the external defibrillator 800. For example, the housing 852 encloses the detection circuit 810, the processor(s) 812, the memory 814, the charging circuit 826, the transceiver(s) 844, or any combination thereof. In some cases, the input device(s) 818 and output device(s) 820 extend from an interior space at least partially surrounded by the housing 852 through a wall of the housing 852. In various examples, the housing 852 acts as a barrier to moisture, electrical interference, and/or dust, thereby protecting various components in the external defibrillator 800 from damage.

In some implementations, the external defibrillator 800 is an automated external defibrillator (AED) operated by an untrained user (e.g., a bystander, layperson, etc.) and can be operated in an automatic mode. In automatic mode, the processor(s) 812 automatically identifies a rhythm in the ECG signal, makes a decision whether to administer a defibrillation shock, charges the capacitor(s) 834, discharges the capacitor(s) 834, or any combination thereof. In some cases, the processor(s) 812 controls the output device(s) 820 to output (e.g., display) a simplified user interface to the untrained user. For example, the processor(s) 812 refrains from causing the output device(s) 820 to display a waveform of the ECG signal and/or the impedance signal to the untrained user, in order to simplify operation of the external defibrillator 800.

In some examples, the external defibrillator 800 is a monitor-defibrillator utilized by a trained user (e.g., a clinician, an emergency responder, etc.) and can be operated in a manual mode or the automatic mode. When the external defibrillator 800 operates in manual mode, the processor(s) 812 cause the output device(s) 820 to display a variety of information that may be relevant to the trained user, such as waveforms indicating the ECG data and/or impedance data, notifications about detected heart rhythms, and the like.

EXAMPLE CLAUSES

The following clauses provide various examples of implementations of the present disclosure:

1. A defibrillator, including: sensors configured to detect electrical activity of a heart of a subject; an input device; a display configured to: present a dynamic real-time snapshot of an electronic electrocardiogram (ECG) indicative of the electrical activity; move to a dynamic historical snapshot of the ECG; and move the dynamic historical snapshot of the ECG to a static historical snapshot of the ECG; and a processor configured to: generate the dynamic real-time snapshot of the ECG, the dynamic historical snapshot of the ECG, and the static historical snapshot of the ECG; identify, via a first swipe received as input from a user to the input device, a first command to move from the dynamic real-time snapshot of the ECG to the dynamic historical snapshot of the ECG; and identify, via a second swipe received as input from the user to the input device, a second command to move from the dynamic historical snapshot of the ECG to the static historical snapshot of the ECG, the dynamic historical snapshot of the ECG being separated from the dynamic real-time snapshot of the ECG by a delay in time that is fixed, the static historical snapshot of the ECG being separated from the dynamic real-time snapshot of the ECG by a delay in time that is continually extending.

2. The defibrillator of clause 1, wherein in response to the processor identifying the first command, the display is further configured to scroll from the dynamic real-time snapshot of the ECG to the dynamic historical snapshot of the ECG.

3. The defibrillator of clause 1 or 2, wherein the processor is further configured to generate, as the dynamic historical snapshot of the ECG, a dynamic 12-lead historical snapshot of the ECG, and wherein the display is further configured to move from the dynamic real-time snapshot of the ECG to the dynamic 12-lead historical snapshot of the ECG.

4. The defibrillator of any of clauses 1 to 3, wherein the processor is further configured to generate, as the static historical snapshot of the ECG, a pseudo-paper snapshot that is static and includes historical data, and wherein the display is further configured to move from the dynamic historical snapshot of the ECG to the pseudo-paper snapshot of the ECG.

5. A medical device, including: sensors configured to detect electrical activity of a heart of a subject; an input device; a display configured to: present a dynamic real-time snapshot of an electronic electrocardiogram (ECG) indicative of the electrical activity; and move from the dynamic real-time snapshot of the ECG to a historical snapshot of the ECG; and a processor configured to: generate the dynamic real-time snapshot of the ECG and the historical snapshot of the ECG, the historical snapshot of the ECG being selected from among a dynamic historical snapshot of the ECG or a static historical snapshot of the ECG; and identify, via input from a user to the input device, a command to move from the dynamic real-time snapshot of the ECG to the historical snapshot of the ECG.

6. The medical device of clause 5, wherein the processor is further configured to identify, via a swipe received as input from the user to the input device, the command, and wherein the display is further configured to scroll to the historical snapshot of the ECG.

7. The medical device of clause 5 or 6, wherein the processor is further configured to generate, as the historical snapshot of the ECG, a 12-lead snapshot of the ECG that is dynamic and includes historical data, wherein the display is further configured to move to the 12-lead snapshot of the ECG.

8. The medical device of any of clauses 5 or 7, wherein the processor is further configured to generate, as the historical snapshot of the ECG, a pseudo-paper snapshot of the ECG that is static and includes historical data, and wherein the display is further configured to move to the pseudo-paper snapshot of the ECG.

9. The medical device of any of clauses 5 to 8, wherein the processor is further configured to generate the historical snapshot of the ECG with a different format from the dynamic real-time snapshot of the ECG.

10. The medical device of any of clauses 5 to 9, wherein the medical device includes a monitor-defibrillator.

11. A method, including: detecting, by sensors of a medical device, electrical activity of a heart of a subject; generating, by a processor of the medical device, a dynamic real-time snapshot of an electronic electrocardiogram (ECG) indicative of the electrical activity, and a historical snapshot of the ECG, the historical snapshot of the ECG being selected from among a dynamic historical snapshot of the ECG or a static historical snapshot of the ECG; presenting, by a display of the medical device, the dynamic real-time snapshot of the ECG; identifying, by the processor, and via input from a user to an input device of the medical device, a command to move from the dynamic real-time snapshot of the ECG to the historical snapshot of the ECG; and moving, by the display, from the dynamic real-time snapshot of the ECG to the historical snapshot of the ECG.

12. The method of clause 11, wherein identifying the command includes identifying, via a swipe received as input from the user to the input device, the command, and wherein moving to the historical snapshot of the ECG includes scrolling, by the display, to the historical snapshot of the ECG.

13. The method of clause 11 or 12, wherein generating the historical snapshot of the ECG includes generating, as the historical snapshot of the ECG, the dynamic historical snapshot of the ECG that includes a 12-lead snapshot of the ECG that is dynamic and includes historical data, wherein moving the historical snapshot of the ECG includes moving, by the display, to the 12-lead snapshot of the ECG.

14. The method of any of clauses 11 to 13, wherein generating the historical snapshot of the ECG includes generating, as the historical snapshot of the ECG, the static historical snapshot of the ECG that includes a pseudo-paper snapshot of the ECG that is static and includes historical data, and wherein moving the historical snapshot of the ECG includes moving, by the display, to the pseudo-paper snapshot of the ECG.

15. The method of any of clauses 11 to 14, wherein generating the historical snapshot of the ECG includes generating the historical snapshot of the ECG with a different format from the dynamic real-time snapshot of the ECG.

16. The method of any of clauses 11 to 15, wherein the historical snapshot of the ECG corresponds to a previous time separated from a current time with which the dynamic real-time snapshot of the ECG is associated, wherein identifying the command includes identifying, via a swipe as received as input from the user to the input device, the command, and wherein moving to the historical snapshot of the ECG includes jumping, by the display, to the historical snapshot of the ECG.

17. The method of any of clauses 11 to 16, wherein identifying the command includes: identifying a first type of swipe having a faster speed or a longer length than a second type of swipe; and identifying, via the second type of swipe received as input from the user to the input device, the command, wherein moving to the historical snapshot of the ECG includes jumping, by the display and in response to identifying the second type of swipe, to the historical snapshot of the ECG.

18. The method of any of clauses 11 to 17, wherein identifying the command includes: identifying a first type of swipe having a faster speed or a longer length than a second type of swipe; and identifying, via the first type of swipe received as input from the user to the input device, the command, and wherein moving to the historical snapshot of the ECG includes scrolling, by the display and in response to identifying the first type of swipe, to the historical snapshot of the ECG.

19. The method of any of clauses 11 to 18, wherein identifying the command includes: identifying a first type of swipe having a faster speed or a longer length than a second type of swipe; and identifying, via a first swipe received as input from the user to the input device, the command that includes a first command, wherein moving includes scrolling, by the display and in response to identifying that the first swipe is the first type, to the historical snapshot of the ECG that includes a first historical snapshot of the ECG, further including: identifying, via a second swipe received as input from the user to the input device, a second command, jumping, by the display and in response to identifying that the second swipe is the second type, to a second historical snapshot of the ECG.

20. The method of any of clauses 11 to 19, further including: storing, by a memory, a log of signals of the ECG; wherein moving to the historical snapshot of the ECG includes jumping, by the display, to the historical snapshot of the ECG, the historical snapshot of the ECG including historical segments of the signals of the ECG being separated from current segments of the signals of the ECG by intermediate segments that increase in size as time passes.

21. A defibrillator, including: sensors configured to detect electrical activity of a heart of a subject; an input device; a display configured to: present a dynamic real-time snapshot of an electronic electrocardiogram (ECG) indicative of the electrical activity; and move from the dynamic real-time snapshot of the ECG to a historical snapshot of the ECG; and a processor configured to: generate the dynamic real-time snapshot of the ECG and the historical snapshot of the ECG, the historical snapshot of the ECG being selected from among a dynamic historical snapshot of the ECG or a static historical snapshot of the ECG; and identify, via input from a user to the input device, a command to move from the dynamic real-time snapshot of the ECG to the historical snapshot of the ECG.

22. The defibrillator of clause 21, wherein the processor is further configured to identify, via a swipe received as input from the user to the input device, the command, and wherein the display is further configured to scroll to the historical snapshot of the ECG.

23. The defibrillator of clause 21 or 22, wherein the processor is further configured to generate, as the historical snapshot of the ECG that includes a dynamic historical snapshot of the ECG, a 12-lead snapshot of the ECG with historical data.

24. The defibrillator of any of clauses 21 to 23, wherein the processor is further configured to generate, as the historical snapshot of the ECG that includes a static historical snapshot of the ECG, a pseudo-paper snapshot of the ECG with historical data, and wherein the display is further configured to move to the pseudo-paper snapshot of the ECG.

25. A medical device, including: two or more sensors configured to detect electrical activity of a heart of a subject; an input device; a display configured to: present a snapshot of an electronic electrocardiogram (ECG) indicative of the electrical activity; in response to identifying a first command, update the snapshot of the ECG when the display is in a backtrack mode; and in response to identifying a second command, update the snapshot of the ECG when the display is in a snapback mode; and a processor configured to: generate the snapshot of the ECG, the snapshot being multi-dimensional; identify, via a first touch received as input from a user to the input device, the first command to update the snapshot of the ECG; identify, via a second touch received as input from the user to the input device, the second command to update the snapshot of the ECG.

26. The medical device of clause 25, wherein the processor is further configured to: prior to generating the snapshot that includes a current snapshot, generate an initial snapshot of the ECG, and an initial timestamp linked to initial data in the initial snapshot of the ECG; and generate a current timestamp linked to current data in the current snapshot of the ECG, and wherein the display is further configured to: update the current snapshot of the ECG to be the initial snapshot of the ECG when the display is in the backtrack mode; and present, when the display is in a historical mode, the initial snapshot of the ECG to which the initial data is linked.

27. The medical device of clause 25 or 26, wherein the processor is further configured to: identify, via a third touch received as input from the user to the input device, a third command to zoom in the snapshot of the ECG when the display is in a pseudo-paper mode, the third touch including a tap, a double tap, or a pinch; and identify, via a fourth touch received as input from the user to the input device of the medical device, a fourth command to pan in the snapshot of the ECG when the display is in the pseudo-paper mode, wherein the display is further configured to: zoom in to a portion of a signal of the ECG in the snapshot of the ECG, the portion being identified by the third touch, and pan to a different portion of the signal of the ECG, the different portion being identified by the fourth touch.

28. The medical device of any of clauses 25 to 27, wherein the processor is further configured to: identify, via a third touch received as input from the user to the input device, a third command to output the snapshot of the ECG, the third touch including a tap at a print button or a save button on the input device, the snapshot of the ECG that is output including a 12-lead snapshot of the ECG.

29. The medical device of any of clauses 25 to 28, wherein the display is further configured to: present, as the snapshot of the ECG, a first 12-lead snapshot of the ECG when the display is in an active mode, the first 12-lead snapshot of the ECG being associated with a current time; and update the snapshot of the ECG when the display is in the backtrack mode to present a second 12-lead snapshot of the ECG when the display is in a historical mode, the second 12-lead snapshot being associated with a previous time.

30. The medical device of any of clauses 25 to 29, wherein the display is further configured to: in response to identifying the first command, scroll the snapshot of the ECG linearly when the display is the backtrack mode; and in response to identifying the second command, jump the snapshot of the ECG non-linearly when the display is in the snapback mode.

31. The medical device of any of clauses 25 to 30, wherein the display is further configured to: in response to identifying the first command, jump the snapshot of the ECG non-linearly when the display is the backtrack mode; and in response to identifying the second command, jump the snapshot of the ECG non-linearly when the display is in the snapback mode.

32. A method, including: detecting, by sensors of a medical device, electrical activity of a heart of a subject; generating a snapshot of an electronic electrocardiogram (ECG) indicative of the electrical activity, the snapshot being multi-dimensional; identifying, by a processor of the medical device, and via a first touch received as input from a user to an input device of the medical device, a first command to update the snapshot of the ECG when a display of the medical device is in a backtrack mode; identifying, via a second touch received as input from the user to the input device, a second command to update the snapshot of the ECG when the display is in a snapback mode; presenting, by the display of the medical device, the snapshot of the ECG; updating, by the display, the snapshot of the ECG when the display is in the backtrack mode; and updating, by the display, the snapshot of the ECG when the display is in the snapback mode.

33. The method of clause 32, wherein the first touch is received when the snapshot of the ECG is presented by the display in an active mode, the first touch including a first type of swipe having an opposing direction, a faster speed, or a longer length than a second type of swipe, and wherein the second touch is received when the snapshot of the ECG is presented by the display in a historical mode, the second touch including a tap or the second type of swipe.

34. The method of clause 32 or 33, wherein updating the snapshot of the ECG in the snapback mode includes jumping from the snapshot having data associated with the ECG at a historical time to the snapshot having data associated with the ECG at a current time.

35. The method of any of clauses 32 to 34, wherein the snapshot that is multi-dimensional includes data associated with the ECG at different times corresponding to temporal dimensions.

36. The method of any of clauses 32 to 35, wherein the snapshot when the display is in a historical mode includes a static historical snapshot, and wherein a first period of time between a previous time and a backtrack time associated with the snapshot when the display is initially in the historical mode is less than a second period of time between a current time and a snapback time associated with the snapshot when the display begins operating in the snapback mode.

37. The method of any of clauses 32 to 36, wherein the snapshot when the display is in a historical mode includes a dynamic historical snapshot, and wherein a first period of time between a previous time and a backtrack time associated with the snapshot when the display is initially in the historical mode is equal to a second period of time between a current time and a snapback time associated with the snapshot when the display begins operating in the snapback mode.

38. The method of any of clauses 32 to 37, wherein the second touch is received when the display is in a historical mode, the second touch including a tap at a snapback button presented by the display.

39. The method of any of clauses 32 to 38, wherein the first touch includes a first type of swipe having a different direction, a different speed, or a different length than a second type of swipe, and wherein the second touch includes the second type of swipe.

40. The method of any of clauses 32 to 38, further including: identifying, via a third touch received as input from the user to the input device, a third command to zoom in the snapshot of the ECG when the display is in a pseudo-paper mode, the third touch including a tap, a double tap, or a pinch; and zooming in, by the display, to a portion of a signal of the ECG in the snapshot of the ECG, the portion being identified by the tap, the double tap, or the pinch.

41. A medical device, including: sensors configured to detect physiological parameters of a subject; an input device; a display configured to: present a dynamic real-time snapshot of an physiological parameter indicative of physiological activity; move to a dynamic historical snapshot of the physiological parameter; and move the dynamic historical snapshot of the physiological parameter to a static historical snapshot of the physiological parameter; and a processor configured to: generate the dynamic real-time snapshot of the physiological parameter, the dynamic historical snapshot of the physiological parameter, and the static historical snapshot of the physiological parameter; identify, via a first swipe received as input from a user to the input device, a first command to move from the dynamic real-time snapshot of the physiological parameter to the dynamic historical snapshot of the physiological parameter; and identify, via a second swipe received as input from the user to the input device, a second command to move from the dynamic historical snapshot of the physiological parameter to the static historical snapshot of the physiological parameter, the dynamic historical snapshot of the physiological parameter being separated from the dynamic real-time snapshot of the physiological parameter by a delay in time that is fixed, the static historical snapshot of the physiological parameter being separated from the dynamic real-time snapshot of the physiological parameter by a delay in time that is continually extending.

42. The medical device of clause 41, wherein the physiological parameter is end-tidal $CO_2$, $SpO_2$ plethysmography, blood pressure, or temperature.

43. The medical device of clause 42, wherein in response to the processor identifying the first command, the display is further configured to scroll from the dynamic real-time snapshot of the physiological parameter to the dynamic historical snapshot of the physiological parameter.

44. The medical device of any of clauses 41 to 43, wherein the processor is further configured to generate, as the static historical snapshot of the physiological parameter, a pseudo-paper snapshot that is static and includes historical data, and wherein the display is further configured to move from the dynamic historical snapshot of the physiological parameter to the pseudo-paper snapshot of the physiological parameter.

CONCLUSION

The features disclosed in the foregoing description, or the following claims, or the accompanying drawings, expressed in their specific forms or in terms of a means for performing the disclosed function, or a method or process for attaining the disclosed result, as appropriate, may, separately, or in any combination of such features, be used for realizing implementations of the disclosure in diverse forms thereof.

As will be understood by one of ordinary skill in the art, each implementation disclosed herein can comprise, consist essentially of or consist of its particular stated element, step, or component. Thus, the terms "include" or "including" should be interpreted to recite: "comprise, consist of, or consist essentially of." The transition term "comprise" or "comprises" means has, but is not limited to, and allows for the inclusion of unspecified elements, steps, ingredients, or components, even in major amounts. The transitional phrase "consisting of" excludes any element, step, ingredient or component not specified. The transition phrase "consisting essentially of" limits the scope of the implementation to the specified elements, steps, ingredients or components and to those that do not materially affect the implementation. As used herein, the term "based on" is equivalent to "based at least partly on," unless otherwise specified.

Unless otherwise indicated, all numbers expressing quantities, properties, conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range, i.e. denoting somewhat more or somewhat less than the stated value or range, to within a range of $\pm 20\%$ of the stated value; $\pm 19\%$ of the stated value; $\pm 18\%$ of the stated value; $\pm 17\%$ of the stated value; $\pm 16\%$ of the stated value; $\pm 15\%$ of the stated value; $\pm 14\%$ of the stated value; $\pm 13\%$ of the stated value; $\pm 12\%$ of the stated value; $\pm 11\%$ of the stated value; $\pm 10\%$ of the stated value; $\pm 9\%$ of the stated value; $\pm 8\%$ of the stated value; $\pm 7\%$ of the stated value; $\pm 6\%$ of the stated value; $\pm 5\%$ of the stated value; $\pm 4\%$ of the stated value; $\pm 3\%$ of the stated value; $\pm 2\%$ of the stated value; or $\pm 1\%$ of the stated value.

Notwithstanding that the numerical ranges and parameters setting forth the broad scope of the disclosure are approximations, the numerical values set forth in the specific examples are reported as precisely as possible. Any numerical value, however, inherently contains certain errors necessarily resulting from the standard deviation found in their respective testing measurements.

The terms "a," "an," "the" and similar referents used in the context of describing implementations (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein is intended merely to better illuminate implementations of the disclosure and does not pose a limitation on the scope of the disclosure. No language in the specification should be construed as indicating any non-claimed element essential to the practice of implementations of the disclosure.

Groupings of alternative elements or implementations disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Certain implementations are described herein, including the best mode known to the inventors for carrying out implementations of the disclosure. Of course, variations on these described implementations will become apparent to those of ordinary skill in the art upon reading the foregoing description. The inventors expect skilled artisans to employ such variations as appropriate, and the inventors intend for implementations to be practiced otherwise than specifically described herein. Accordingly, the scope of this disclosure includes all modifications and equivalents of the subject matter recited in the claims appended hereto as permitted by applicable law. Moreover, any combination of the above-described elements in all possible variations thereof is encompassed by implementations of the disclosure unless otherwise indicated herein or otherwise clearly contradicted by context.

**Claims**

1. A defibrillator, comprising:

sensors configured to detect electrical activity of a heart of a subject;
an input device;
a display configured to:

present a dynamic real-time snapshot of an electronic electrocardiogram (ECG) indicative of the electrical activity;
move to a dynamic historical snapshot of the ECG; and
move the dynamic historical snapshot of the ECG to a static historical snapshot of the ECG; and
a processor configured to:

generate the dynamic real-time snapshot of the ECG, the dynamic historical snapshot of the ECG, and the static historical snapshot of the ECG;
identify, via a first swipe received as input from a user to the input device, a first command to move from the dynamic real-time snapshot of the ECG to the dynamic historical snapshot of the ECG; and
identify, via a second swipe received as input from the user to the input device, a second command to move from the dynamic historical snapshot of the ECG to the static historical snapshot of the ECG, the dynamic historical snapshot of the ECG being separated from the dynamic real-time snapshot of the ECG by a delay in time that is fixed, the static historical snapshot of the ECG being separated from the dynamic real-time snapshot of the ECG by a delay in time that is continually extending.

2. The defibrillator of claim 1, wherein in response to the processor identifying the first command, the display is further configured to scroll from the dynamic real-time snapshot of the ECG to the dynamic historical snapshot of the ECG.

3. The defibrillator of claim 1 or 2, wherein the processor is further configured to generate, as the dynamic historical snapshot of the ECG:

a dynamic 12-lead historical snapshot of the ECG, and the display is further configured to move from the dynamic real-time snapshot of the ECG to the dynamic 12-lead historical snapshot of the EC; and/or.
a pseudo-paper snapshot that is static and comprises historical data, and the display is further configured to move from the dynamic historical snapshot of the ECG to the pseudo-paper snapshot of the ECG.

4. A medical device, comprising:

sensors configured to detect electrical activity of a heart of a subject;
an input device;
a display configured to:

present a dynamic real-time snapshot of an electronic electrocardiogram (ECG) indicative of the electrical activity; and
move from the dynamic real-time snapshot of the ECG to a historical snapshot of the ECG; and

a processor configured to:

generate the dynamic real-time snapshot of the ECG and the historical snapshot of the ECG, the historical snapshot of the ECG being selected from among a dynamic historical snapshot of the ECG or a static historical snapshot of the ECG; and
identify, via input from a user to the input device, a command to move from the dynamic real-time snapshot of the ECG to the historical snapshot of the ECG,

wherein the medical device optionally comprises a monitor-defibrillator.

5. The medical device of claim 4, wherein the processor is further configured to identify, via a swipe received as input from the user to the input device, the command, and
wherein the display is further configured to scroll to the historical snapshot of the ECG.

6. The medical device of claim 4 or 5, wherein the processor is further configured to generate, as the historical snapshot of the ECG:

a 12-lead snapshot of the ECG that is dynamic and comprises historical data, and the display is further configured to move to the 12-lead snapshot of the ECG, and/or.
a pseudo-paper snapshot of the ECG that is static and comprises historical data, and the display is further configured to move to the pseudo-paper snapshot of the ECG,

wherein the historical snapshot of the ECG and the dynamic real-time snapshot of the ECG have different formats.

7. A method, comprising:

detecting, by sensors of a medical device, electrical activity of a heart of a subject;
generating, by a processor of the medical device, a dynamic real-time snapshot of an electronic electrocardiogram (ECG) indicative of the electrical activity, and a historical snapshot of the ECG, the historical snapshot of the ECG being selected from among a dynamic historical snapshot of the ECG or a static historical snapshot of the ECG;
presenting, by a display of the medical device, the dynamic real-time snapshot of the ECG;
identifying, by the processor, and via input from a user to an input device of the medical device, a command to move from the dynamic real-time snapshot of the ECG to the historical snapshot of the ECG; and
moving, by the display, from the dynamic real-time snapshot of the ECG to the historical snapshot of the ECG.

8. The method of claim 7, wherein identifying the command comprises identifying, via a swipe received as input from the user to the input device, the command, and
wherein moving to the historical snapshot of the ECG comprises scrolling, by the display, to the historical snapshot of the ECG.

9. The method of claim 7 or 8, wherein generating the historical snapshot of the ECG comprises generating, as the historical snapshot of the ECG:

the dynamic historical snapshot of the ECG that comprises a 12-lead snapshot of the ECG that is dynamic and comprises historical data, and moving the historical snapshot of the ECG comprises moving, by the display, to the 12-lead snapshot of the ECG; and/or
the static historical snapshot of the ECG that comprises a pseudo-paper snapshot of the ECG that is static and comprises historical data, and the historical snapshot of the ECG comprises moving, by the display, to the pseudo-paper snapshot of the ECG.

10. The method of claim 7 or 8, wherein generating the historical snapshot of the ECG comprises generating the historical snapshot of the ECG with a different format from the dynamic real-time snapshot of the ECG.

11. The method of any of claims 7 to 10, wherein the historical snapshot of the ECG corresponds to a previous time separated from a current time with which the dynamic real-time snapshot of the ECG is associated,

wherein identifying the command comprises identifying, via a swipe as received as input from the user to the input device, the command, and
wherein moving to the historical snapshot of the ECG comprises jumping, by the display, to the historical snapshot of the ECG.

12. The method of any of claims 7-11, wherein identifying the command comprises:

identifying a first type of swipe having a faster speed or a longer length than a second type of swipe; and
identifying, via the second type of swipe received as input from the user to the input device, the command,
wherein moving to the historical snapshot of the ECG comprises jumping, by the display and in response to identifying the second type of swipe, to the historical snapshot of the ECG.

13. The method of any of claims 7 to 12, wherein identifying the command comprises:

identifying a first type of swipe having a faster speed or a longer length than a second type of swipe; and

identifying, via the first type of swipe received as input from the user to the input device, the command, and wherein moving to the historical snapshot of the ECG comprises scrolling, by the display and in response to identifying the first type of swipe, to the historical snapshot of the ECG.

14. The method of any of claims 7 to 12, wherein identifying the command comprises:

identifying a first type of swipe having a faster speed or a longer length than a second type of swipe; and identifying, via a first swipe received as input from the user to the input device, the command that comprises a first command,
wherein moving comprises scrolling, by the display and in response to identifying that the first swipe is the first type, to the historical snapshot of the ECG that comprises a first historical snapshot of the ECG,
further comprising:

identifying, via a second swipe received as input from the user to the input device, a second command, jumping, by the display and in response to identifying that the second swipe is the second type, to a second historical snapshot of the ECG.

15. The method of any of claims 7 to 14, further comprising:

storing, by a memory, a log of signals of the ECG;
wherein moving to the historical snapshot of the ECG comprises jumping, by the display, to the historical snapshot of the ECG, the historical snapshot of the ECG comprising historical segments of the signals of the ECG being separated from current segments of the signals of the ECG by intermediate segments that increase in size as time passes.

FIG. 1

FIG. 2

FIG. 3

ELECTROCARDIOGRAM (ECG) 124

HISTORICAL ECG 126

USER INTERFACE (UI) 104

75

36

98

75

36

98

120
80

LIVE VIEW

SWIPE INPUT 400

INDICATOR 404

TAP INPUT 402

EP 4 778 462 A1

FIG. 4

500

DETECT, BY SENSORS OF A MEDICAL DEVICE, ELECTRICAL ACTIVITY OF A HEART OF A SUBJECT
502

GENERATE, BY A PROCESSOR OF THE MEDICAL DEVICE, A DYNAMIC REAL-TIME SNAPSHOT OF AN ELECTRONIC ELECTROCARDIOGRAM (ECG) INDICATIVE OF THE ELECTRICAL ACTIVITY, AND A HISTORICAL SNAPSHOT OF THE ECG, THE HISTORICAL SNAPSHOT OF THE ECG BEING SELECTED FROM AMONG A DYNAMIC HISTORICAL SNAPSHOT OF THE ECG OR A STATIC HISTORICAL SNAPSHOT OF THE ECG
504

PRESENT, BY A DISPLAY OF THE MEDICAL DEVICE, THE DYNAMIC REAL-TIME SNAPSHOT OF THE ECG
506

IDENTIFY, BY THE PROCESSOR, AND VIA INPUT FROM A USER TO AN INPUT DEVICE OF THE MEDICAL DEVICE, A COMMAND TO MOVE FROM THE DYNAMIC REAL-TIME SNAPSHOT OF THE ECG TO THE HISTORICAL SNAPSHOT OF THE ECG
508

MOVE, BY THE DISPLAY, FROM THE DYNAMIC REAL-TIME SNAPSHOT OF THE ECG TO THE HISTORICAL SNAPSHOT OF THE ECG
510

# FIG. 5

600 ⤳

DETECT, BY SENSORS OF A MEDICAL DEVICE, ELECTRICAL ACTIVITY OF A
HEART OF A SUBJECT
602

GENERATE A SNAPSHOT OF AN ELECTRONIC ELECTROCARDIOGRAM (ECG)
INDICATIVE OF THE ELECTRICAL ACTIVITY, THE SNAPSHOT BEING MULTI-
DIMENSIONAL
604

IDENTIFY, BY A PROCESSOR OF THE MEDICAL DEVICE, AND VIA A FIRST
TOUCH RECEIVED AS INPUT FROM A USER TO AN INPUT DEVICE OF THE
MEDICAL DEVICE, A FIRST COMMAND TO UPDATE THE SNAPSHOT OF THE
ECG WHEN A DISPLAY OF THE MEDICAL DEVICE IS IN A BACKTRACK MODE
606

IDENTIFY, VIA A SECOND TOUCH RECEIVED AS INPUT FROM THE USER TO
THE INPUT DEVICE, A SECOND COMMAND TO UPDATE THE SNAPSHOT OF
THE ECG WHEN THE DISPLAY IS IN A SNAPBACK MODE
608

PRESENT, BY THE DISPLAY OF THE MEDICAL DEVICE, THE SNAPSHOT OF
THE ECG
610

UPDATE, BY THE DISPLAY, THE SNAPSHOT OF THE ECG WHEN THE
DISPLAY IS IN THE BACKTRACK MODE
612

UPDATE, BY THE DISPLAY, THE SNAPSHOT OF THE ECG WHEN THE
DISPLAY IS IN THE SNAPBACK MODE
614

# FIG. 6

BATTERY CONDITION 728

HISTORICAL CAPNOGRAM 726

USER INTERFACE (UI) 104

LIVE VIEW

INDICATOR 704

TAP INPUT 702

FIG. 7

BATTERY CONDITION 728

CAPNOGRAM 724

SENSOR 710

SWIPE INPUT 700

40 20 0

800

INDIVIDUAL
808

ECG
ELECTRODES
806

ECG
LEADS
804

ECG PORT
802

DEFIBRILLATION
ELECTRODES
838

DEFIBRILLATION
LEADS
840

800

DEFIBRILLATION
PORT
842

DISCHARGE
SWITCH(ES)
836

DETECTION
CIRCUIT 810

CAPACITOR(S)
834

CHARGING
SWITCH(ES)
832

DISCHARGE
CIRCUIT 828

POWER
SOURCE
830

OUTPUT
DEVICE(S) 820

CHARGING CIRCUIT
826

INPUT DEVICE(S)
818

PROCESSOR(S)
812

HOUSING
852

MEMORY 814

DETECTOR
816

ADVISOR
822

INITIATOR
824

COORDINATOR
850

TRANSCEIVER(S)
844

COMMUNICATION
NETWORK(S)
846

EXTERNAL
DEVICE(S)
848

FIG. 8

## EUROPEAN SEARCH REPORT

**Application Number**

EP 26 15 2433

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/049776 A1 (FREEMAN GARY A [US] ET AL) 16 February 2023 (2023-02-16) * figures 1B, 7, 10A, B * * paragraphs [0037], [0039], [0040], [0057], [0101], [0105], [0171], [0241] * | 1-15 | INV. A61B5/339 A61B5/00 |

-----

**TECHNICAL FIELDS SEARCHED (IPC)**

A61B

The present search report has been drawn up for all claims

| Place of search | Date of completion of the search | Examiner |
|---|---|---|
| The Hague | 29 May 2026 | Schwenke, Stephanie |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 26 15 2433

This annex lists the patent family members relating to the patent documents cited in  the above-mentioned European search report.
The members are as contained in the European Patent Office EDP file on
The European Patent Office is in no way liable for these particulars which are merely given for the purpose of information.

29-05-2026

| Patent document cited in search report | Publication date | Patent family member(s) | | Publication date |
|---|---|---|---|---|
| US 2023049776 A1 | 16-02-2023 | CN | 113939794 A | 14-01-2022 |
| | | EP | 3942395 A1 | 26-01-2022 |
| | | EP | 4513508 A2 | 26-02-2025 |
| | | US | 2023049776 A1 | 16-02-2023 |
| | | US | 2025046408 A1 | 06-02-2025 |
| | | WO | 2020197903 A1 | 01-10-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63746890